# EUROPEAN PATENT APPLICATION

(11) **EP 2 546 358 A1**
(43) Date of publication of application: **16.01.2013**
(21) Application number: 11382243.1
(22) Date of filing: 15.07.2011
(51) Int. Cl.: C12Q 1/68, C12Q 1/70

(54) **Methods and reagents for efficient control of HIV progression**

(71) Applicant: Laboratorios del. Dr. Esteve, S.A., 08041 Barcelona (ES); Fundació Privada Institut de Recerca de la SIDA-Caixa, 08916 Badalona (ES)
(72) Inventor: Arnedo Valero, Mireia, 08172 Sant Cugat del Valles (ES); Plana Prades, Monserrat, 08009 Barcelona (ES); Gatell Artigas, Josep Maria, 08016 Barcelona (ES)
(74) Representative: ABG Patentes, S.L.

(57) **Abstract**

The invention relates to methods for the identification of HIV-infected patients capable of controlling viral load based on the determination of the expression levels of different miRNAs. In addition, the invention also relates to methods for controlling HIV infection by using some of the differentially expressed miRNAs or polynucleotides encoding said miRNAs as well as to compositions comprising a miRNA or a polynucleotide encoding said miRNA and an anti-HIV agent.

## Description

### FIELD OF THE INVENTION

The invention relates to the field of immunology and, in particular, to methods for the identification of patients capable of controlling HIV progression as well as to the identification of specific miRNAs associated to HIV progression control and the uses thereof for improving the immunological response of HIV patients.

### BACKGROUND OF THE INVENTION

The infection by the Human Immunodefiency Virus (VIH-1) is characterized, during the natural history of the disease, either by an increased plasmatic viremia and by a progressive loss of CD4+ T lymphocytes which will involve, in absence of antiretroviral therapy an important immunologic impairment with the consequent appearance of oportunistic infections as well other related events and lastly the death. The appearance of the highly active antiretroviral therapy (HAART) produced dramatic changes in the progression and evolution of the HIV-1 infection. Nevertheless, the inability of this treatment for eliminate the latent HIV-1 in the integrated proviral DNA, and the persistence of cryptic and low levels of viral replication which allow the cell-to-cell transmission, as well as the need of an strict adherence, and the toxicity of the compounds, continue to be enormous limitations for the accomplishment for life of this therapy.

On the other side, even if in the majority of HIV-1 infected individuals the viral replication causes the progressive destruction of the immune system and the fatal evolution to AIDS, there are a low proportion of individuals immunologically "privileged", the long term non progressors (LTNP), who are characterized by stable and maintained CD4 T cell counts and by the presence of a great and conserved anti-HIV-1 T cell immune response (Th, CTL) and neutralizing antibodies. These responses are associated with control of the viral replication showing a plasma viral load, without any treatment, very low or undetectable, fact that could not be attributed to a defective virus, to protective genetic variants of the HIV-1 coreceptors or of the chemokines these receptors bind and inhibit the infection. Among this group of LTNP, less than 1% of individuals are also able to maintain a plasma viral load below detectable limits (<50 copies/ml): they are the called elite controllers (EC) (Lambote O et al. Clin. Infect. Dis., 2005, 41:1053-1056 and Grabar S et al. AIDS 2009, 23:1163-1169). The attractive of this group of special patients is that they would allow define parameters involved in the control of viral replication, fact of major relevance for the future development of effective treatment and vaccines. The viremia control by the host could be due in part to viral factors, or immunological parameters or genetic ones.

A great number of studies have tried to dissect host factors, mostly the differences in the antiviral immune response, responsible of the viral replication control in these individuals. In this sense, it has been described that the presence of an earlier and effective innate immune response could be important. More precisely, EC seem to maintain both the normal phenotype of NK cells and the preserved cytotoxic capacity of these cells. It is not well known if this is a relevant ability for the control of the viral replication but it could also be postulated that NK cells could act in conjunction with dendritic cells at an early time of the appropriated adaptive immune response and in the modulation of the inflammatory phenomenon observed during the HIV-1 pathogenic infection. Similarly to what occurred in pathogenic models of SIV infection in natural hosts, EC have usually less depletion of CD4+ T cells in mucosa and lower levels of bacterial translocation than normal progressors, fact that could be partially related to a better type I IFN and other inflammatory cytokines production by plasmacytoid dendritic cells, having consequently a moderated level of chronic activation of the immune system.

Concerning to humoral immunity, usually EC do not show greater titers of broad range neutralizing antibodies when compared to chronic progressors, suggesting that the viremia control could not be attributed to the protective role of this arm of the immune response.

For instance, the findings more contundent seem to be related to the relevant role of the cellular immune reponse in the control of the HIV-1 infection. In addition to the finding that EC present a great number of HIV-1-specific CD8+ T cells, it is important to note the best quality of these cells associated to their polyfunctionality (production of IFN-gamma, TNF-alpha, IL-2, MIP-1beta; cytotoxic activity, proliferative capacity...), both in periphery and mucosa (14). It has been shown that the greater production of cytolytic enzymes (granzyme B, perforin) is the main feature that allows the reduction of viral replication in autologous CD4+ T cells from these kind of patients, fact that is not observed in HIV-+ patients HAART- treated and with suppressed viremia.

The importance of the response of CD8+ T cells in EC is also clear from the fact that these individuals may have an over-expression of certain HLA class I alleles described as protectors, such as HLA B*57, B*27 and as a dominant response to Gag protein epitopes. Despite all this evidence, it is now clear that the CTL response is not quite enough to explain the control of viral replication in all EC, since there is some heterogeneity in this group of patients with these characteristics.

Regarding CD4+ T cells, even if they not show any special resistance to infection, it has been described that EC have an increased number of anti-HIV+ CD4+T cells with an increased proliferative capacity of these cells, either dependent on IL-2 or as a consequence of a reduction of the pool of exhausted cells, as evidenced by the fact that the inhibitory molecule CTLA-4 is not overexpressed in the EC's HIV-specific CD4 T cells, as occurs in progressors or after HAART therapy.

Finally, another factor involved in the desirable control of viral replication could be the existence of a balance between the protective antiviral immune response of CD4 and CD8 T cells and the modulation mechanisms exerted by regulatory T cells (Tregs) This could minimize the nonspecific and undesirable activation phenomena. In this sense, it has been described in EC the presence of high levels of Tregs that correlate with a low level of CD4+ T cell activation.

A better understanding of the causes of the differences in VL could provide pointers to new vaccines and drugs that control the virus.

### SUMMARY OF THE INVENTION

In a first aspect, the invention relates to a method for the identification of a controller HIV-infected patient which comprises the determination of the level of one or more miRNAs selected from the group consisting of miR-27a, miR-27b, miR-29b, miR-221 miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-Sp, miR-191 and/or miR-197 in a sample from said patient,
wherein increased expression level of one or more of the miR-27a, miR-27b, miR-29b and/or miR-221 miRNAs with respect to a reference value is indicative that the patient is a HIV controller or
wherein decreased expression levels of one or more of miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-5p, miR-191 and/or miR-197 with respect to a reference value is indicative that the patient is a HIV controller.

In a second aspect, the invention relates to a miRNAs selected from the group consisting of miR-27a, miR-27b, miR-29b, miR-125, miR-146, miR-155 and miR-221 for use in the treatment or prevention of a disease caused by HIV.

In a third asprct, the invention relates to a microbiocide composition or kit-of-parts comprising
(i) A miRNAs selected from the group consisting of miR-27a, miR-27b, miR-29b, miR-125, miR-146, miR-155 and miR-221 or a polynucleotide encoding said miRNA and
(ii) an anti-HIV agent.

In a fourth aspect, the invention relates to a kit comprising reagents adequate for the determination of the expression levels of one or more miRNAs selected from the group consisting of miR-27a, miR-27b, miR-29b, miR-221.

### DESCRIPTION OF THE FIGURES

**Figure 1****.** Study groups: miRNA isolated from 8 million of PBMC activated with PHA were compared between these groups: 1) Elite controllers (HIV patients with VL<200 cp/ml without treatment); 2) Viremic Progressors (HIV patients with VL>5000 cp/ml without treatment); 3) ART (HIV patients with VL<200 cp/ml during HAART); 4) HIV negative
**Figure 2****.** Hierarchical clustering of differentially expressed miRNAs between Elite Controllers, Viremic Progressors and patients with ART.
**Figure 3****.** Mean fold change EC and VP versus HIV negative. This figure shows the log2 of fold change (-ΔCt) of the 23 most differentially expressed miRNA comparing Elite Controllers and Viremic Progressors versus HIV negative. The dotted line indicates a 2-fold difference in expression compared with the mean of expression of all PBMCs.
**Figure 4****.** Relative expression of indicated 22 miRNA expression comparing elite controllers and ART versus HIV negative.
**Figure 5****.** Relative expression of indicated 22 miRNA expression comparing elite controllers and ART versus viremic progressors.

### DETAILED DESCRIPTION OF THE INVENTION

### Method for the identification of HIV controller patients

The present invention is based on the surprising finding that the expression levels of different miRNAs differs in HIV-infected patients which are elite controllers with respect to patients which show viremic progression or patients wherein viremic progression is controlled by the use of antiretroviral therapy. Thus, in a first aspect, the invention relates to a method for the identification of a controller HIV-infected patient which comprises the determination of the level of one or more miRNAs selected from the group consisting of miR-27a, miR-27b, miR-29b, miR-221 miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-Sp, miR-191 and/or miR-197 in a sample from said patient,
wherein increased expression level of one or more of the miR-27a, miR-27b, miR-29b and/or miR-221 miRNAs with respect to a reference value is indicative that the patient is a HIV controller or
wherein decreased expression levels of one or more of miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-5p, miR-191 and/or miR-197 with respect to a reference value is indicative that the patient is a HIV controller.

The term "identification", as used herein refers to methods by which the skilled artisan can estimate and even determine whether or not a subject is suffering from a given disease or condition, e.g. whether the subject is a HIV controller. The skilled artisan often makes a diagnosis on the basis of one or more diagnostic indicators, such as for example a biomarker (e.g., a miRNA or a set of miRNAs), the amount (including presence or absence) of which is indicative of the presence, severity, or absence of the condition. It also relates to evaluating the probability according to which a subject suffers from a disease. As it will be understood by persons skilled in the art, such evaluation, although it is preferred that it is, normally may not be correct for 100 percent of the subjects to be diagnosed. The term, however, requires that a statistically significant part of the subjects can be identified as suffering from the disease or having a predisposition for it. The person skilled in the art can determine if a part is statistically significant without further delay by using several well known statistic evaluation tools, for example, determination of confidence intervals, determination of the p value, Student's t-test, Mann-Whitney test, etc. The details are in Dowdy and Wearden, Statistics for Research, John Wiley and Sons, New York 1983. The preferred confidence intervals are of at least 50 percent, at least 60 percent, at least 70 percent, at least 80 percent, at least 90 percent, at least 95 percent. The p values are preferably 0.2, 0.1, 0.05.

As used herein a "controller" refers to a subject infected with HIV that exhibits a decrease in HIV viral load after the individual is infected with HIV and maintains the decreased HIV viral load over time. A "controller" also refers to an HIV-infected subject who remains asymptomatic with normal CD4 positive T-cell counts and low or undetectable plasma viral loads despite having never been treated with antiretroviral medications. HIV infected individuals that are controllers are capable of maintaining their viral load at a very low levels, for example plasma HIV RNA levels < 2000 copies/mL in the absence of antiretroviral therapy, measured three times over a period spanning at least 12 months.

Features of controllers as defined by the HIV Controller Consortium are:
- Maintain HIV RNA levels below 2000 copies/mL
- No antiretroviral therapy for 1 year or longer
- Episodes of viremia are acceptable as long as they represent the minority of all available determinations

In a preferred embodiment, the controller is an elite controller. As used herein, the term "elite controller" refers to HIV-infected patients which show HIV RNA below the level of detection in the absence of antiviral therapy for the respective available ultrasensitive assay (e.g., < 75 copies per ml by bDNA or < 50 copies per ml by ultrasensitive PCR). Criteria for identifying elite controllers have been described by Goudsmit et al. (AIDS, 2002, 16:791 -793).

Features of Elite Controllers as defined by the HIV Controller Consortium are:
- Maintain HIV RNA levels below 50 copies/mL
- No antiretroviral therapy for 1 year or longer
- Episodes of viremia are acceptable as long as there are not consecutive episodes

In a first step, the method for the identification of HIV controller patients according to the present invention comprises the determination of the level of one or more of a miRNA selected from the group consisting of miR-27a, miR-27b, miR-29b, miR-221 miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-Sp, miR-191 and/or miR-197 miR-27a, miR-27b, miR-29b and miR-221 in a sample from said patient.

As used herein, the term "miRNA", also known as microRNA, refers to double-stranded RNA molecules, typically of about 21-23 nucleotides in length, which regulate gene expression. miRNAs are encoded by genes that are transcribed from DNA but not translated into protein (non-coding RNA); instead they are processed from primary transcripts known as pri-miRNA to short stem-loop structures called pre-miRNA and finally to functional miRNA. Following processing, mature miRNAs are incorporated into a RISC (RNA-Induced Silencing Complex), which participates in RNA interference (RNAi). miRNAs can pair with target mRNAs that contain sequences only partially complementary (e.g., 30 percent , 35 percent , 40 percent , 50 percent , 55 percent , 60 percent , 65 percent , 70 percent , 75 percent , 80 percent or more) to the miRNA. Such pairing results in repression of mRNA translation without altering mRNA stability. Alternatively, miRNAs with a substantial degree of complementarity to their targets effect gene silencing by mediating mRNA degradation (Hutvagner and Zamore (2002) Science 297:2056-2060). As expression of precursor microRNAs (i.e., pri-miRNAs) is often developmentally regulated, miRNAs are often referred to interchangeably in the art as "small temporal RNAs" or "stRNAs".

The term "miR-27a", "miR-27b", "miR-29b", "miR-221", "miR-155", "miR-146a", "miR-484", "miR-339", "miR-186", "miR-106a", "miR-17", "miR-590", "miR-374b", "miR-140-Sp", "miR-16", "miR-454", "miR-200c", "miR-146b", "miR-200b", "miR-422a", "miR-125a-Sp", "miR-191" and/or "miR-197" as used herein, refer to the human miRNAs as defined in the miRBase:: Sequences" database of the Wellcome Trust Sanger Institute (http://microrna.sanger.ac.uk/sequences/index.shtml). The nucleic acid sequences corresponding to the miRNAs, hairpin pre-miRNAs and miRNA* s described in Tables 1-3 are available from the "miRBase:: Sequences" database of the Wellcome Trust Sanger Institute (http://microrna.sanger.ac.uk/sequences/index.shtml). miRNA, hairpin miRNA, and miRNA* sequence information can be found in Release 17 (April 2011). This database is described further in the following papers, incorporated by reference herein in their entirety: Griffiths-Jones S. et al., Nucleic Acids Res., 36 (Database lssue):D154-D158; Griffiths-Jones S. et al., Nucleic Acids Res., 2006, 34 (Database Issue):D140-D144 and Griffiths-Jones S. et al., Nucleic Acids Res., 2004, 32 (Database Issue) :D109-D111. Nomenclature conventions used to describe miRNA sequences are described further in Ambros V et al., RNA, 2003, 9:277-279, the entire contents of which are incorporated herein by reference.

**Table 1 Candidate miRNA comparing EC with VP**

| Detector-miRNA | p-value | Adj. P (FDR)* (Benjamini/Hochberg) | Mature Sequence | SEQ ID NO: |
|---|---|---|---|---|
| Hsa-miR-454 | 7.78E-04 | 0,034755975 | UAGUGCAAUAUUGCUUAUAGGGU | 1 |
| Hsa-miR-590-5p | 0,001131326 | 0,034755975 | GAGCUUAUUCAUAAAAGUGCAG | 2 |
| Hsa-miR-146b-5p | 0,001629186 | 0,034755975 | UGAGAACUGAAUUCCAUAGGCU | 3 |
| Hsa-miR-200c | 0,001629186 | 0,034755975 | UAAUACUGCCGGGUAAUGAUGGA | 4 |
| Hsa-miR-422a | 0,001629186 | 0,034755975 | ACUGGACUUAGGGUCAGAAGGC | 5 |
| Hsa-miR-27b | 0,001629186 | 0,034755975 | UUCACAGUGGCUAAGUUCUGC | 6 |
| Hsa-miR-27a | 0,002322095 | 0,037153512 | UUCACAGUGGCUAAGUUCCGC | 7 |
| Hsa-miR-16 | 0,002322095 | 0,037153512 | UAGCAGCACGUAAAUAUUGGCG | 8 |
| Hsa-miR-221 | 0,003275898 | 0,0044963185 | AGCUACAUUGUCUGCUGGGUUUC | 9 |
| Hsa-miR-191 | 0,00457444 | 0,0044963185 | CAACGGAAUCCCAAAAGCAGCUG | 10 |
| Hsa-miR-374b | 0,00457444 | 0,0044963185 | AUAUAAUACAACCUGCUAAGUG | 11 |
| Hsa-miR-29b | 0,00457444 | 0,0044963185 | UAGCACCAUUUGAAAUCAGUGUU | 12 |
| Hsa-miR-339-3p | 0,00457444 | 0,0044963185 | UCCCUGUCCUCCAGGAGCUCACG | 13 |
| Hsa-miR-146a | 0,006322948 | 0,0044963185 | UGAGAACUGAAUUCCAUGGGUU | 14 |
| Hsa-miR-17 | 0,006322948 | 0,0044963185 | CAAAGUGCUUACAGUGCAGGUAG | 15 |
| Hsa-miR-125a-5p | 0,006322948 | 0,0044963185 | UCCCUGAGACCCUUUAACCUGUGA | 16 |
| Hsa-miR-106a | 0,006322948 | 0,0044963185 | AAAAGUGCUUACAGUGCAGGUAG | 17 |
| Hsa-miR-200b | 0,006322948 | 0,04814771 | UAAUACUGCCUGGUAAUGAUGA | 18 |
| Hsa-miR-484 | 0,008651542 | 0,04814771 | UCAGGCUCAGUCCCCUCCCGAU | 19 |
| Hsa-miR-155 | 0,008651542 | 0,04814771 | UUAAUGCUAAUCGUGAUAGGGGU | 20 |
| Hsa-miR-140-5p | 0,008651542 | 0,04814771 | CAGUGGUUUUACCCUAUGGUAG | 21 |
| Hsa-miR-197 | 0,008651542 | 0,04814771 | UUCACCACCUUCUCCACCCAGC | 22 |
| Hsa-miR-186 | 0,008651542 | 0,04814771 | CAAAGAAUUCUCCUUUUGGGCU | 23 |

| | | | | |
|---|---|---|---|---|
| 5% of False Discovery Range (FDR) | | | | |

The method of the invention require determining miRNA expression levels in a cell or in a biological sample. Methods for determining miRNA expression levels in cells or biological samples include generic methods for the detection and quantification of nucleic acids especially RNA, optimized methods for the detection and quantification of small RNA species, as both mature and precursor miRNAs fall into this category as well as specially designed methods for the detection and quantification of miRNA species. Illustrative, non-limitative, examples of methods which can be used to facilitate the testing of multiple miRNAs include:
1. Methods based in hybridization such as Northern blot analysis and in situ hybridization
2. Multiplex and/or singleplex real-time RT-PCR (reagents available from, e.g., Applied Biosystems and System Biosciences (SBI)), including quantitative real time reverse transcriptase PCR (qRT-PCR) as described in US 5,928,907 and US 6,015,674;
3. Single-molecule detection as described by Neely, et al., Nat. Methods. 3(1):41-6 (2006) and in US 6,355,420; US 6,916,661; and US 6,632,526;
4. Bead-based flow cytometric methods as described by Lu, et al., Nature 435:7043 (2005) and in US 6,524,793; and
5. Assays using arrays of nucleic acids as described by Nelson, et al., Nat. Methods 1(2):155-61 (2004); Wu, et al., RNA 13(1):151-9 (2007) and in US 6,057,134; US 6,891,032; US 7,122,303; US 6,458,583; US 6,465,183; US 6,461,816; US 6,458,583; US 7,026,124; US 7,052,841; US 7,060,809; US 6,436,640; and US 7,060,809.

Total cellular RNA can be purified from cells by homogenization in the presence of nucleic acid extraction buffer, followed by centrifugation. Nucleic acids are precipitated, and DNA is removed by treatment with DNase and precipitation. Nucleic acids, especially RNA and specifically miRNA can be isolated using any techniques known in the art. There are two main methods for isolating RNA: (i) phenol-based extraction and (ii) silica matrix or glass fiber filter (GFF)-based binding. Phenol-based reagents contain a combination of denaturants and RNase inhibitors for cell and tissue disruption and subsequent separation of RNA from contaminants. Phenol-based isolation procedures can recover RNA species in the 10-200-nucleotide range e.g., miRNAs, 5S rRNA, 5.8S rRNA, and U1 snRNA. If a sample of "total" RNA was purified by the popular silica matrix column or GFF procedure, it may be depleted in small RNAs. Extraction procedures such as those using Trizol or TriReagent, however will purify all RNAs, large and small, and are the recommended methods for isolating total RNA from biological samples that will contain miRNAs/siRNAs. Any method required for the processing of a sample prior to quantifying the level of the miRNAs according to the method of the invention falls within the scope of the present invention. These methods are typically well known by a person skilled in the art.

RNA molecules can be separated by gel electrophoresis on agarose gels according to standard techniques, and transferred to nitrocellulose filters by, e.g., the so- called "Northern Blot" technique. The RNA is then immobilized on the filters by heating. Detection and quantification of specific RNA is accomplished using appropriately labeled DNA or RNA probes complementary to the RNA in question (see, for example, Molecular Cloning: A Laboratory Manual, J. Sambrook et ai, eds., 2n Edition, Cold Spring Harbor Laboratory Press, 1989, Chapters 10 and 11, the disclosures of which are incorporated herein by reference.

Suitable probes for Northern blot hybridization of a given miRNA gene product can be produced using the nucleotide sequence of an miRNA. miRNA, hairpin pre-miRNA and miRNA* sequences known in the art from the miRBase:: Sequences database.

Methods for preparation of labeled DNA and RNA probes, and the conditions for hybridization thereof to target nucleotide sequences, are described in Molecular Cloning: A Laboratory Manual, J. Sambrook et al., eds., 2nd edition, Cold Spring Harbor Laboratory Press, 1989. Chapters 10 and 11, the disclosures of which are herein incorporated by reference. For example, the nucleic acid probe can be labeled with, e.g., a radionuclide such as 3H, 32P, 33P, 14C, or 35S; a heavy metal; or a ligand capable of functioning as a specific binding pair member for a labeled ligand (e.g., biotin, avidin or an antibody), a fluorescent molecule, a chemiluminescent molecule, an enzyme or the like. Probes can be labeled to high specific activity by either the nick translation method of Rigby et al. (Rigby (1977), /. Mol. Biol. 113:237-251), or by the random priming method of Fienberg et al. (Fienberg (1983), Anal. Biochem. 132:6-13, the entire disclosures of which are herein incorporated by reference. The latter is the method of choice for synthesizing 32P-labeled probes of high specific activity from single-stranded DNA or from RNA templates. For example, by replacing preexisting nucleotides with highly radioactive nucleotides according to the nick translation method, it is possible to prepare "P-labeled nucleic acid probes with a specific activity well in excess of 10 cpm/microgram. Autoradiographic detection of hybridization can then be performed by exposing hybridized filters to photographic film. Densitometric scanning of the photographic films exposed by the hybridized filters provides an accurate measurement of miRNA gene transcript levels. Using another approach, miRNA gene transcript levels can be quantified by computerized imaging systems, such the Molecular Dynamics 400- B 2D Phosphorimagcr available from Amersham Biosciences, Piscataway, NJ.

Where radionuclide labeling of DNA or RNA probes is not practical, the random-primer method can be used to incorporate an analogue, for example, the dTTP analogue 5-(N-(N -biotinyl-epsilon-aminocaproyl)-3-aminoallyl)deoxyuridine triphosphate, into the probe molecule. The biotinylated probe oligonucleotide can be detected by reaction with biotin-binding proteins, such as avidin, streptavidin, and antibodies (e.g., anti-biotin antibodies) coupled to fluorescent dyes or enzymes that produce color reactions. In addition to Northern and other RNA blotting hybridization techniques, determining the levels of RNA transcripts can be accomplished using the technique of in situ hybridization. This technique requires fewer cells than the Northern blotting technique, and involves depositing whole cells onto a microscope cover slip and probing the nucleic acid content of the cell with a solution containing radioactive or otherwise labeled nucleic acid (e.g., cDNA or RNA) probes. This technique is particularly well-suited for analyzing tissue biopsy samples from subjects. The practice of the in situ hybridization technique is described in more detail in U.S. Pat. No. 5,427,916, the entire disclosure of which is incorporated herein by reference. Suitable probes for in situ hybridization of a given miRNA gene product can be produced using the nucleotide sequence of an miRNA. miRNA, miRNA* and pre-miRNA hairpin sequences known in the art correspond to the miRNAs, miRNA*s and pre-miRNA hairpins described in the miRBase:: Sequences.

The relative number of miRNA gene transcripts in cells can also be determined by reverse transcription of miRNA gene transcripts, followed by amplification of the reverse-transcribed transcripts by polymerase chain reaction (RT-PCR).

The levels of miRNA gene transcripts can be quantified in comparison with an internal standard, for example, the level of mRNA from a "housekeeping" gene present in the same sample. A suitable "housekeeping" gene for use as an internal standard includes, e.g., myosin or glyceraldehyde-3-phosphate dehydrogenase (GAPDH). The methods for quantitative RT-PCR and variations thereof are within the skill in the art.

The three commonly used methods of quantitative polymerase chain reaction are through agarose gel electrophoresis, the use of SYBR Green (a double stranded DNA dye), and the fluorescent reporter probe. The latter two of these three can be analysed in real-time, constituting real-time polymerase chain reaction method.

Using SYBR Green dye gives results in real time. A DNA binding dye binds all newly synthesized double stranded (ds)DNA and an increase in fluorescence intensity is measured, thus allowing initial concentrations to be determined. However, SYBR Green will label all dsDNA including any unexpected PCR products as well as primer dimers, leading to potential complications and artefacts. The reaction is prepared as usual, with the addition of fluorescent dsDNA dye. The reaction is run, and the levels of fluorescence are monitored; the dye only fluoresces when bound to the dsDNA. With reference to a standard sample or a standard curve, the dsDNA concentration in the PCR can be determined.

The fluorescent reporter probe method is the most accurate and most reliable of the methods. It uses a sequence-specific nucleic acid based probe so as to only quantify the probe sequence and not all double stranded DNA. It is commonly carried out with DNA based probes with a fluorescent reporter and a quencher held in adjacent positions, so-called dual-labelled probes. The close proximity of the reporter to the quencher prevents its fluorescence; it is only on the breakdown of the probe that the fluorescence is detected. This process depends on the 5' to 3' exonuclease activity of the polymerase involved. The real-time quantitative PCR reaction is prepared with the addition of the dual-labelled probe. On denaturation of the double-stranded DNA template, the probe is able to bind to its complementary sequence in the region of interest of the template DNA (as the primers will too). When the PCR reaction mixture is heated to activate the polymerase, the polymerase starts synthesizing the complementary strand to the primed single stranded template DNA. As the polymerisation continues it reaches the probe bound to its complementary sequence, which is then hydrolysed due to the 5'-3' exonuclease activity of the polymerase thereby separating the fluorescent reporter and the quencher molecules. This results in an increase in fluorescence, which is detected. During thermal cycling of the real-time PCR reaction, the increase in fluorescence, as released from the hydrolysed dual-labelled probe in each PCR cycle is monitored, which allows accurate determination of the final, and so initial, quantities of DNA.

Any method of PCR that can determine the expression of a nucleic acid molecule as defined herein falls within the scope of the present invention. A preferred embodiment of the present invention regards the real-time quantitative RT-PCR method, based on the use of either SYBR Green dye or a dual-labelled probe for the detection and quantification of nucleic acids according to the herein described.

In some embodiments, it is desirable to simultaneously determine the expression level of a plurality of different of miRNAs in a sample. In certain instances, it may be desirable to determine the expression level of the transcripts of the miRNAs in a plurality of samples. Assessing expression levels for hundreds of miRNAs is time consuming and requires a large amount of total RNA (at least 20 micro g for each Northern blot) and autoradiographic techniques that require radioactive isotopes. To overcome these limitations, an oligolibrary in microchip format may be constructed containing a set of probe oligonucleotides specific for a set of miRNA genes

The set of mature miRNAs, miRNA*s, and pre-miRNA hairpin precursors known in the art at the time of filing of the instant application may be found in Tables 1 - 3. The nucleic acid sequences corresponding to the miRNAs, hairpin pre-miRNAs and rniRNA*s described in Tables 1-3 are available from the "miRBase: Sequences" database of the Wellcome Trust Sanger Institute (http://microrna.sanger.ac.uk/sequences/index.shtml). The microchip is prepared from gene-specific oligonucleotide probes generated from known miRNAs. According to one embodiment, the array contains two different oligonucleotide probes for each miRNA, one containing the active sequence and the other being specific for the precursor of the miRNA. The array may also contain controls such as one or more (e.g. mouse) sequences differing from (e.g. human) orthologs by only a few bases, which can serve as controls for hybridization stringency conditions. tRNAs from both species may also be printed on the microchip, providing an internal, relatively stable positive control for specific hybridization. One or more appropriate controls for non-specific hybridization may also be included on the microchip. For this purpose, sequences are selected based upon the absence of any homology with any known miRNAs.

The microchip may be fabricated by techniques known in the art. For example, probe oligonucleotides of an appropriate length, e.g., 40 nucleotides, are 5'-amine modified at position C6 and printed using commercially available microarray systems, e.g., the GeneMachine OmniGrid™ 100 Microarrayer and Amersham CodeLink™ activated slides. Labeled cDNA oligomer corresponding to the target RNAs is prepared by reverse transcribing the target RNA with labeled primer. Following first strand synthesis, the RNA/DNA hybrids are denatured to degrade the RNA templates. The labeled target cDN?s thus prepared are then hybridized to the microarray chip under hybridizing conditions, e.g. 6 X SSPE/30 percent formamide at 25 °C Centigrade for 18 hours, followed by washing in 0.75 X TNT at 37 °C for 40 minutes. At positions on the array where the immobilized probe DNA recognizes a complementary target cDNA in the sample, hybridization occurs. The labeled target cDNA marks the exact position on the array where binding occurs, allowing automatic detection and quantification. The output consists of a list of hybridization events, indicating the relative abundance of specific cDNA sequences, and therefore the relative abundance of the corresponding complementary miRNAs, in the sample. According to one embodiment, the labeled cDNA oligomer is a biotin-labeled cDNA, prepared from a biotin-labeled primer. The microarray is then processed by direct detection of the biotin-containing transcripts using, e.g., Streptavidin-Alexa647 conjugate, and scanned utilizing conventional scanning methods. The intensity of each spot on the array is proportional to the abundance of the corresponding miRNA in the sample.

The use of the array has several advantages for miRNA expression detection. First, the global expression of several hundred genes can be identified in a single sample at one time point. Second, through careful design of the oligonucleotide probes, expression of both mature and precursor molecules can be identified. Third, in comparison with Northern blot analysis, the chip requires a small amount of RNA, and provides reproducible results using 2.5 micro g of total RNA. The relatively limited number of miRNAs (a few hundred per species) potentially allows the construction of a common microarray for several species, with distinct oligonucleotide probes for each. Such a tool would allow for analysis of trans-species expression for each known miRNA under various conditions. The microarray methods described herein are useful for both the identification of miRNA signatures and the diagnostic and therapeutic applications of the invention.

In the method of the invention, cells or biological samples as described above can further comprise suitable controls. Such suitable controls will be obvious to one skilled in the art and are considered part of the common knowledge. The relative miRNA expression in the control or normal samples can further be determined with respect to one or more RNA expression standards. The standards can comprise, for example, a zero miRNA gene expression level, the miRNA gene expression level in a standard cell line, or the average level of miRNA gene expression previously obtained for a population of normal human controls.

The term "biological sample", as used herein, refers to a small part of a subject, representative of the whole and may be constituted by a biopsy or a body fluid sample. Biopsies are small pieces of tissue and may be fresh, frozen or fixed, such as formalin-fixed and paraffin embedded (FFPE). Body fluid samples may be blood, plasma, serum, urine, sputum, cerebrospinal fluid, milk, or ductal fluid samples and may likewise be fresh, frozen or fixed. Samples may be removed surgically, by extraction i.e. by hypodermic or other types of needles, by microdissection or laser capture. The sample should contain any biological material suitable for detecting the desired biomarker (miRNA), thus, said sample should, advantageously comprise cell material from the subject. Examples of the biological sample include whole blood, PBMC, and T cells. Methods for collecting and preparing these biological samples are known in the art. In a particular embodiment, the sample is a body fluid sample such as a blood sample; preferably, the sample comprises peripheral blood mononuclear cells (PBMC) from the subj ect.

In a second step, the method for the identification of HIV controller patients comprises comparing the expression levels of one or more of the above miRNAs with reference values for each miRNA, wherein increased expression level of said miRNA with respect to a reference value is indicative that the patient is a HIV controller

The term "reference value" as used herein, refers to the expression level of the miRNA under consideration in a reference sample. A "reference sample", as used herein, means a sample obtained from subjects, preferably two or more subjects, known to be free of the disease or, alternatively, from the general population. The suitable reference expression levels of miRNAs can be determined by measuring the expression levels of said miRNAs in several suitable subjects, and such reference levels can be adjusted to specific subject populations. In a preferred embodiment, the reference sample is obtained from a viremic progressor or from a HIV-infected patient undergoing ART.

Chronic progressor HIV infected individuals exhibit a high viral load, for example plasma HIV RNA levels> 10,000 copies/mL, as compared to an individual that is not infected with HIV. The viral load of a chronic progressor increases over time, for example months (for example 1, 2, 3, 4, 5, 6, 7, 8, 9, 10, 11 or 12 months) or years (for example 2, 3, 4, 5, 6, 7, 8, 9, 10 years).
n
Alternatively, it is possible that the reference sample is obtained by pooling samples from viremic progressors or by pooling samples from patients which are undergoing ART. The expression profile of the miRNAs in the reference sample can, preferably, be generated from a population of two or more subjects; for example, the population can comprise 3, 4, 5, 10, 15, 20, 30, 40, 50 or more subjects.

HIV-infected patient undergoing ART refers to a HIV-infected patient wherein the viral is low by means of the administration of antiretroviral therapy (ART). The term "antiretroviral therapy" is meant to refer to the administration of one or more antiretroviral drugs to inhibit the replication of HIV combination therapy. Typically, ART involves the administration of at least one antiretroviral agent (or, commonly, a cocktail of antiretrovirals) such as nucleoside reverse transcriptase inhibitor (e.g., zidovudine (AZT, lamivudine (3TC) and abacavir), non-nucleoside reverse transcriptase inhibitor (e.g., nevirapine and efavirenz), and protease inhibitor (e.g., indinavir, ritonavir and lopinavir). The term Highly Active Antiretroviral Therapy (HAART) refers to treatment regimens designed to aggressively suppress viral replication and progress of HIV disease, usually consisting of three or more different drugs, such as for example, two nucleoside reverse transcriptase inhibitors and a protease inhibitors.

As used herein, "increased" as it refers to the level of miRNA of a subject means at least about 1-fold greater (for example 1, 2, 3, 4, 5, 10, 20, 30, 40, 50, 60, 70, 80, 90, 100, 1000, 10,000-fold or more) than the reference value. "Increased" as it refers to the viral load of a subject also means at least about 5 percent greater (for example 5, 6, 7, 8, 9, 10, 15, 20, 25, 30, 35, 40, 45, 50, 55, 60, 65, 70, 75, 80, 85, 90, 95, 99 or 100 percent) than the level in the reference sample or with respect to the reference value for said miRNA.

As used herein, "decreased" as it refers to viral load of a subject means at least about 1-fold (for example 1, 2, 3, 4, 5, 10, 20, 30, 40, 50, 60, 70, 80, 90, 100, 1000, 10,000-fold or more) less than the viral load of a control subject. "Decreased" as it refers to the viral load of a subject also means at least about 5 percent less than (for example 5, 6, 7, 8, 9, 10, 15, 20, 25, 30, 35, 40, 45, 50, 55, 60, 65, 70, 75, 80, 85, 90, 95, 99 or 100 percent) the viral load of a control subject.

### Therapeutic uses of the miRNAs

In yet another aspect, the invention relates to a miRNAs selected from the group consisting of miR-27a, miR-27b, miR-29b, miR-125, miR-146, miR-155 and miR-221 or a polynucleotide encoding said miRNA for use in the treatment or prevention of a disease caused by HIV. Alternatively, the invention relates to the use of a miRNAs selected from the group consisting of miR-27a, miR-27b, miR-29b, miR-125, miR-146, miR-155 and miR-221 or a polynucleotide encoding said miRNA for the manufacture of a medicament for the treatment of a disease caused by HIV. Alternatively, the invention relates to a method for the treatment of a disease caused by HIV in a subject in need thereof comprising the administration to said subject a composition comprising a a miRNAs selected from the group consisting of miR-27a, miR-27b, miR-29b, miR-125, miR-146, miR-155 and miR-221 or a polynucleotide encoding said miRNA.

The term "treatment", as used anywhere herein comprises any type of therapy, which aims at terminating, preventing, ameliorating and/or reducing the susceptibility to a clinical condition as described herein. In a preferred embodiment, the term treatment relates to prophylactic treatment (i.e. a therapy to reduce the susceptibility of a clinical condition, a disorder or condition as defined herein).

Thus, "treatment," "treating," and the like, as used herein, refer to obtaining a desired pharmacologic and/or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse affect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, and/or immune deficiency.

The terms "prevent," "preventing," and "prevention", as used herein, refer to a decrease in the occurrence of pathological cells in an animal. The prevention may be complete (e.g. the total absence of pathological cells in a subject). The prevention may also be partial, such that for example the occurrence of pathological cells in a subject is less than that which would have occurred without the present invention. Prevention also refers to reduced susceptibility to a clinical condition.

The term disease caused by HIV-1 infection, as used herein, refers to any disease which results from the infection by HIV of one or more cellular types. Most preferably, the disease is selected from the group consisting of Acquired Immune Deficiency Syndrome (AIDS) or the HIV- or HAART-associated disorders HIV-associated dementia (HAD), Immune Reconstitution Disease (IRD) and lipodystrophy.

The term "miRNA" has been defined above in detail and is used with the same meaning in the context of the present invention.

The term "polynucleotide encoding a miRNAs" is a polynucleotide comprising a mature sequence of the one or more miRNAs. The polynucleotide may comprise the sequence encoding the pri-miRNA or pre-miRNA sequence for the one or more miRNAs. The polynucleotide comprising the mature, pre-miRNA, or pri-miRNA sequence can be single stranded or double stranded. The polynucleotides may contain one or more chemical modifications, such as locked nucleic acids, peptide nucleic acids, sugar modifications, such as 2'-O-alkyl (e.g. 2'-O-methyl, 2'-O-methoxyethyl), 2'-fluoro, and 4' thio modifications, and backbone modifications, such as one or more phosphorothioate, morpholino, or phosphonocarboxylate linkages. In some embodiments, the polynucleotide comprising one or more miRNA sequences is conjugated to a steroid, such as cholesterol, a vitamin, a fatty acid, a carbohydrate or glycoside, a peptide, or another small molecule ligand. In certain embodiments, an agonist of one or more miRNAs is an agent distinct from the miRNA itself that acts to increase, supplement, or replace the function of the one or more miRNAs.

In some embodiments, the DNA molecule encoding the miRNA or precursor thereof is found in an expression cassette.

The expression cassette comprises one or more regulatory sequences, selected on the basis of the cells to be used for expression, operably linked to a polynucleotide encoding the miRNA or precursor thereof. "Operably linked" is intended to mean that the nucleotide sequence of interest (i.e., a DNA encoding a microRNA or precursor thereof) is linked to the regulatory sequence(s) in a manner that allows for expression of the nucleotide sequence (e.g., in an in vitro transcription/translation system or in a cell when the expression cassette or vector is introduced into a cell). "Regulatory sequences" include promoters, enhancers, and other expression control elements (e.g., polyadenylation signals). See, for example, Goeddel (1990) in Gene Expression Technology: Methods in Enzymology 185 (Academic Press, San Diego, California). Regulatory sequences include those that direct constitutive expression of a nucleotide sequence in many types of host cells and those that direct expression of the nucleotide sequence only in certain host cells (e.g., tissue-specific regulatory sequences). It will be appreciated by those skilled in the art that the design of the expression cassette can depend on such factors as the choice of the host cell to be transformed, the level of expression of the miRNA or precursor thereof, and the like. Such expression cassettes typically include one or more appropriately positioned sites for restriction enzymes, to facilitate introduction of the nucleic acid into a vector.

It will further be appreciated that appropriate promoter and/or regulatory elements can readily be selected to allow expression of the relevant miRNA or precursor thereof in the cell of interest. In certain embodiments, the promoter utilized to direct intracellular expression of a miRNA or precursor thereof is a promoter for RNA polymerase III (Pol III). References discussing various Pol III promoters, include, for example, Yu et al (2002) Proc. Natl. Acad. Set 99(9), 6047-6052; Sui et al. (2002) Proc. Natl Acad. Sci. 99(8), 5515-5520 (2002); Paddison et al. (2002) Genes and Dev. 16, 948-958; Brummelkamp et al. (2002) Science 296, 550-553; Miyagashi (2002) Biotech. 20, 497-500; Paul et al. (2002) Nat. Biotech. 20, 505-508; Tuschl et al. (2002) Nat. Biotech. 20, 446-448, each of which is herein incorporated by reference in its entirety. According to other embodiments, a promoter for RNA polymerase I, e.g., a tRNA promoter, can be used. See McCown et al (2003) Virology 313(2):514-24; Kawasaki (2003) Nucleic Acids Res. 31 (2):700-7, each of which is herein incorporated by reference in its entirety. The regulatory sequences can also be provided by viral regulatory elements.

For example, commonly used promoters are derived from polyoma, Adenovirus 2, cytomegalovirus, and Simian Virus 40. For other suitable expression systems for both prokaryotic and eukaryotic cells, see Chapters 16 and 17 of Sambrook et al. (1989) Molecular Cloning: A Laboratory Manual (2d ed., Cold Spring Harbor Laboratory Press, Plainview, New York). See, Goeddel (1990) in Gene Expression Technology: Methods in Enzymology 185 (Academic Press, San Diego, California), In vitro transcription can be performed using a variety of available systems including the T7, SP6, and T3 promoter/polymerase systems (e.g., those available commercially from Promega, Clontech, New England Biolabs, and the like). Vectors including the T7, SP6, or T3 promoter are well known in the art and can readily be modified to direct transcription of miRNAs or precursors thereof. When double-stranded miRNA precursors are synthesized in vitro, the strands can be allowed to hybridize before introducing into a cell or before administration to a subject. As noted above, miRNAs or precursors thereof can be delivered or introduced into a cell as a single RNA molecule including self-complementary portions (e.g., an shRNA that can be processed intracellularly to yield a miRNA), or as two strands hybridized to one another. In other embodiments, the miRNAs or precursors thereof are transcribed in vivo. As discussed elsewhere herein, regardless of if the miRNA or precursor thereof is transcribed in vivo or in vitro, in either scenario, a primary transcript can be produced which can then be processed (e.g., by one or more cellular enzymes) to generate the miRNA that accomplishes gene inhibition.

The microbiocide compositions are suitable for preventing or reducing symptoms associated with HIV infection. These include symptoms associated with the minor symptomatic phase of HIV infection, including, for instance, shingles, skin rash and nail infection, mouth sores, recurrent nose and throat infection, and weight loss. In addition, further symptoms associated with the major symptomatic phase of HIV infection, include, for example, oral and vaginal thrush (Candida), persistent diarrhea, weight loss, persistent cough, reactivated tuberculosis, and recurrent herpes infections, such as cold sores (herpes simplex). Symptoms of full-blown AIDS which can be treated in accordance with the present invention, include, for instance, diarrhea, nausea and vomiting, thrush and mouth sores, persistent, recurrent vaginal infections and cervical cancer, persistent generalized lymphadenopathy (PGL), severe skin infections, warts and ringworm, respiratory infections, pneumonia, especially *Pneumocystis carinii pneumonia* (PCP), herpes zoster (or shingles), nervous system problems, such as pains, numbness or "pins and needles" in the hands and feet, neurological abnormalities, Kaposi's sarcoma, lymphoma, tuberculosis, and other opportunistic infections. Beneficial effects of the miRNAs or polynucleotides encoding said miRNAs include, for example, preventing or delaying initial infection of an individual exposed to HIV; reducing viral burden in an individual infected with HIV; prolonging the asymptomatic phase of HIV infection; maintaining low viral loads in HIV infected patients whose virus levels have been lowered via anti-retroviral therapy (ART); increasing levels of CD4 T cells or lessening the decrease in CD4 T cells, both HIV-1 specific and non-specific, in drug naive patients and in patients treated with ART, increasing overall health or quality of life in an individual with AIDS; and prolonging life expectancy of an individual with AIDS. A clinician can compare the effect of immunization with the patient's condition prior to treatment, or with the expected condition of an untreated patient, to determine whether the treatment is effective in inhibiting AIDS.

All forms of HIV-I are potentially treatable with compounds of the present invention. The compounds of the present invention are useful for treating protease inhibitor resistant HIV, reverse transcriptase inhibitor resistant HIV, and entry/fusion inhibitor resistant HIV. These compounds are also useful in the treatment of HIV groups M, N, and O; HIV-I subtypes, including the A1, A2, B, C, D, F1, F2, G, H, and J subtypes; and circulating recombinant HIV forms. The compounds of the present invention are useful for treating CCR5 tropic HIV strains as well as CXCR4 tropic HIV strains.

The miRNA or the polynucleotide according to the invention may further comprise a pharmaceutically acceptable carrier.

A "pharmaceutically acceptable carrier," "pharmaceutically acceptable diluent," or "pharmaceutically acceptable excipient", or "pharmaceutically acceptable vehicle," used interchangeably herein, refer to a non-toxic solid, semisolid or liquid filler, diluent, encapsulating material or formulation auxiliary of any conventional type. A pharmaceutically acceptable carrier is essentially non-toxic to recipients at the dosages and concentrations employed, and is compatible with other ingredients of the formulation. For example, the carrier for a formulation containing polypeptides would not normally include oxidizing agents and other compounds that are known to be deleterious to polypeptides. Suitable carriers include, but are not limited to water, dextrose, glycerol, saline, ethanol, and combinations thereof. The carrier can contain additional agents such as wetting or emulsifying agents, pH buffering agents, or adjuvants which enhance the effectiveness of the formulation. Adjuvants could for example be selected from the group consisting of: AlK(SO4)2, AlNa(SO4)2, AlNH4 (SO4), silica, alum, Al(OH)3, Ca3(PO4)2, kaolin, carbon, aluminum hydroxide, muramyl dipeptides, N-acetyl-muramyl-L-threonyl-D-isoglutamine (thr-DMP), N-acetyl-nornuramyl-L-alanyl-D-isoglutamine (CGP 11687, also referred to as nor-MDP), N-acetylmuramyul-L-alanyl-D-isoglutaminyl-L-alanine-2-(1 '2'-dipalmitoyl-sn - glycero-3-hydroxphosphoryloxy)-ethylamine (CGP 19835A, also referred to as MTP-PE), RIBI (MPL+TDM+CWS) in a 2 percent squalene/Tween-80^{®}emulsion, lipopolysaccharides and its various derivatives, including lipid A, Freund's Complete Adjuvant (FCA), Freund's Incomplete Adjuvants, Merck Adjuvant 65, polynucleotides (for example, poly IC and poly AU acids), wax D from Mycobacterium, tuberculosis, substances found in *Corynebacterium parvum*, *Bordetella pertussis*, and members of the genus Brucella, Titermax, ISCOMS, Quil A, ALUN, Lipid A derivatives, choleratoxin derivatives, HSP derivatives, LPS derivatives, synthetic peptide matrixes or GMDP, interleukin 1, interleukin 2, Montanide ISA-51 and QS-21, CpG oligonucleotide, poly I:C and GM-CSF. *See* Hunter R, US 5,554,372, and Jager E, Knuth A, WO1997028816.

The microbiocide composition can be administered by any means known to one skilled in the art, such as by intramuscular, subcutaneous or intravenous injection, and oral, nasal, or anal administration. *See* Banga A, "Parenteral Controlled Delivery of Therapeutic Peptides and Proteins," in Therapeutic Peptides and Proteins (Technomic Publishing Co., Inc., Lancaster, PA, USA, 1995). To extend the time during which the peptide or protein is available to stimulate a response, the peptide or protein can be provided as an implant, an oily injection, or as a particulate system. The particulate system can be a microparticle, a microcapsule, a microsphere, a nanocapsule, or similar particle. *See* Banga, 1995, *supra*. A particulate carrier based on a synthetic polymer has been shown to act as an adjuvant to enhance the immune response, in addition to providing a controlled release. Aluminum salts can also be used as adjuvants to produce an immune response.

Microbiocide compositions can be formulated in unit dosage form, suitable for individual administration of precise dosages. In pulse doses, a bolus administration of an immunogenic composition that includes a disclosed immunogen is provided, followed by a time-period wherein no disclosed immunogen is administered to the subject, followed by a second bolus administration. A therapeutically effective amount of an immunogenic composition can be administered in a single dose, or in multiple doses, for example daily, during a course of treatment. In specific, non-limiting examples, pulse doses of an immunogenic composition that include a disclosed immunogen are administered during the course of a day, during the course of a week, or during the course of a month.

Microbiocide compositions can be administered whenever the effect (such as decreased signs, symptom, or laboratory results of HIV-1 infection) is desired. Generally, the dose is sufficient to treat or ameliorate symptoms or signs of disease without producing unacceptable toxicity to the subject. Systemic or local administration can be utilized.

Amounts effective for therapeutic use can depend on the severity of the disease and the age, weight, general state of the patient, and other clinical factors. Thus, the final determination of the appropriate treatment regimen will be made by the attending clinician. Typically, dosages used *in vitro* can provide useful guidance in the amounts useful for in situ administration of the pharmaceutical composition, and animal models may be used to determine effective dosages for treatment of particular disorders. *See* Gilman R, et al., Eds., Goodman and Gilman's: The Pharmacological Basis of Therapeutics, 8th Ed. (Pergamon Press, New York, NY, USA, 1990), and Gennaro A, Ed., Remington's Pharmaceutical Sciences, 18th Ed. (Mack Publishing Co., Easton, PA, USA, 1990). Typically, the dose range for a miRNA is from about 0.1 µg/kg body weight to about 100 mg/kg body weight. Other suitable ranges include doses of from about 1 µg/kg to 10 mg/kg body weight. In one example, the dose is about 1.0 µg to about 50 mg, for example, 1 µg to 1 mg, such as 1 mg peptide per subject. The dosing schedule can vary from daily to as seldom as once a year, depending on clinical factors, such as the subject's sensitivity to the peptide and tempo of their disease. Therefore, a subject can receive a first dose of a disclosed therapeutic molecule, and then receive a second dose (or even more doses) at some later time(s), such as at least one day later, such as at least one week later.

The pharmaceutical compositions disclosed herein can be prepared and administered in dose units. Solid dose units include tablets, capsules, transdermal delivery systems, and suppositories. The administration of a therapeutic amount can be carried out both by single administration in the form of an individual dose unit or else several smaller dose units and also by multiple administrations of subdivided doses at specific intervals. Suitable single or divided doses include, but are not limited to about 0.01, 0.1, 0.5, 1, 3, 5, 10, 15, 30, or 50 µg protein/kg/day.

The nucleic acid constructs encoding antigenic the miRNAs described herein are used, for example, in combination, as pharmaceutical compositions (medicaments) for use in therapeutic, for example, prophylactic regimens (such as vaccines) and administered to subjects (e.g. primate subjects, such as human subjects) to elicit an immune response against one or more clade or strain of HIV. For example, the compositions described herein can be administered to a human (or non-human) subject prior to infection with HIV to inhibit infection by or replication of the virus. Thus, the pharmaceutical compositions described above can be administered to a subject to elicit a protective immune response against HIV. To elicit an immune response, a therapeutically effective (e.g. immunologically effective) amount of the nucleic acid constructs are administered to a subject, such as a human (or non-human) subject.

For administration of nucleic acid molecules encoding a miRNA, the nucleic acid can be delivered intracellularly, for example, by expression from an appropriate nucleic acid expression vector which is administered so that it becomes intracellular, such as by use of a retroviral vector, by direct injection, by use of microparticle bombardment (e.g. a gene gun; Biolistic, Dupont), coating with lipids, cell-surface receptors or transfecting agents, or by administering it in linkage to a homeobox-like peptide which is known to enter the nucleus. *See* Morgan J, et al., US 4,980,286, and Joliot A, et al., Proc. Natl. Acad. Sci. USA 1991; 88:1864-1868. The present invention includes all forms of nucleic acid delivery, including synthetic oligos, naked DNA, plasmid and viral, integrated or not into the genome.

In another approach to using nucleic acids, the miRNAs can be expressed by attenuated viral hosts or vectors or bacterial vectors. Recombinant vaccinia virus, adeno-associated virus (AAV), herpes virus, retrovirus, or other viral vectors can be used to express the peptide or protein, thereby eliciting a CTL response.

In one example, a viral vector is utilized. These vectors include, but are not limited to, adenovirus, herpes virus, vaccinia, or an RNA virus such as a retrovirus. In one example, the retroviral vector is a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous Sarcoma Virus (RSV). When the subject is a human, a vector such as the gibbon ape leukemia virus (GaLV) can be utilized. A number of additional retroviral vectors can incorporate multiple genes. All of these vectors can transfer or incorporate a gene for a selectable marker so that transduced cells can be identified and generated. By inserting a nucleic acid sequence encoding a miRNA into the viral vector, along with another gene that encodes the ligand for a receptor on a specific target cell, for example, the vector is now target specific. Retroviral vectors can be made target specific by attaching, for example, a sugar, a glycolipid, or a protein. Preferred targeting is accomplished by using an antibody to target the retroviral vector. Those skilled in the art will know of, or can readily ascertain without undue experimentation, specific polynucleotide sequences which can be inserted into the retroviral genome or attached to a viral envelope to allow target specific delivery of the retroviral vector containing the polynucleotide encoding a miRNA.

Suitable formulations for the nucleic acid constructs, include aqueous and non-aqueous solutions, isotonic sterile solutions, which can contain anti-oxidants, buffers, and bacteriostats, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid carrier, for example, water, immediately prior to use. Extemporaneous solutions and suspensions can be prepared from sterile powders, granules, and tablets. Preferably, the carrier is a buffered saline solution. More preferably, the composition for use in the inventive method is formulated to protect the nucleic acid constructs from damage prior to administration. For example, the composition can be formulated to reduce loss of the adenoviral vectors on devices used to prepare, store, or administer the expression vector, such as glassware, syringes, or needles. The compositions can be formulated to decrease the light sensitivity and/or temperature sensitivity of the components. To this end, the composition preferably comprises a pharmaceutically acceptable liquid carrier, such as, for example, those described above, and a stabilizing agent selected from the group consisting of polysorbate 80, L-arginine, polyvinylpyrrolidone, trehalose, and combinations thereof.

In therapeutic applications, a therapeutically effective amount of the composition is administered to a subject prior to or following exposure to or infection by HIV. When administered prior to exposure, the therapeutic application can be referred to as a prophylactic administration (such as in the form of a vaccine). Single or multiple administrations of the compositions are administered depending on the dosage and frequency as required and tolerated by the subject. In one embodiment, the dosage is administered once as a bolus, but in another embodiment can be applied periodically until a therapeutic result, such as a protective immune response, is achieved. Generally, the dose is sufficient to treat or ameliorate symptoms or signs of disease without producing unacceptable toxicity to the subject. Systemic or local administration can be utilized.

Nucleic acid constructs encoding the miRNAs for use in the present invention can be introduced *in vivo* as naked DNA plasmids. DNA vectors can be introduced into the desired host cells by methods known in the art, including but not limited to transfection, electroporation (e.g. transcutaneous electroporation), microinjection, transduction, cell fusion, DEAE dextran, calcium phosphate precipitation, use of a gene gun, or use of a DNA vector transporter. *See* Wu C, et al., J. Biol. Chem. 1992; 267:963-967, Wu C and Wu G, Biol. Chem. 1988; 263:14621-14624, and Williams R, et al., Proc. Natl. Acad. Sci. USA 1991; 88:2726-2730. Methods for formulating and administering naked DNA to mammalian muscle tissue are also known. *See* Felgner P, et al., US 5,580,859, and US 5,589,466. Other molecules are also useful for facilitating transfection of a nucleic acid *in vivo*, such as cationic oligopeptides, peptides derived from DNA binding proteins, or cationic polymers. *See* Bazile D, et al., WO1995021931 and Byk G, et al., WO1996025508.

Another well known method that can be used to introduce nucleic acid constructs encoding miRNAs into host cells is particle bombardment (aka biolistic transformation). Biolistic transformation is commonly accomplished in one of several ways. One common method involves propelling inert or biologically active particles at cells. *See* Sanford J, et al., US 4,945,050, US 5,036,006, and US 5,100,792.

Alternatively, the vector can be introduced *in vivo* by lipofection. The use of cationic lipids can promote encapsulation of negatively charged nucleic acids, and also promote fusion with negatively charged cell membranes. *See* Felgner P, Ringold G, Science 1989; 337:387-388. Particularly useful lipid compounds and compositions for transfer of nucleic acids have been described. *See* Felgner P, et al., US 5,459,127, Behr J, et al., WO1995018863, and Byk G, WO1996017823.

As with the miRNAs as such, the nucleic acid encoding the miRNA s may be administered in a single dose, or multiple doses separated by a time interval. For example, two doses, or three doses, or four doses, or five doses, or six doses or more can be administered to a subject over a period of several weeks, several months or even several years, to optimize the immune response.

It may be advantageous to administer the microbiocide compositions disclosed herein with other agents such as proteins, peptides, antibodies, and other anti-HIV agents. Examples of such anti-HIV therapeutic agents include nucleoside reverse transcriptase inhibitors, such as abacavir, AZT, didanosine, emtricitabine, lamivudine, stavudine, tenofovir, zalcitabine, zidovudine, and the like, non-nucleoside reverse transcriptase inhibitors, such as delavirdine, efavirenz, nevirapine, protease inhibitors such as amprenavir, atazanavir, indinavir, lopinavir, nelfinavir osamprenavir, ritonavir, saquinavir, tipranavir, and the like, and fusion protein inhibitors such as enfuvirtide and the like. In certain embodiments, immunogenic compositions are administered concurrently with other anti-HIV therapeutic agents. In certain embodiments, the immunogenic compositions are administered sequentially with other anti-HIV therapeutic agents, such as before or after the other agent. One of ordinary skill in the art would know that sequential administration can mean immediately following or after an appropriate period of time, such as hours days, weeks, months, or even years later.

The present invention also contemplates that the administration of the immunogenic compositions according to the invention can be carried out in combination with other suitable therapeutic treatments which are useful for treating a disease caused by infection by HIV. Thus, the invention also relates to a composition or kit-of-parts comprising a immunogenic composition and one or more drugs used for the treatment of a disease caused by HIV infection.

The pharmaceutical composition of the invention may be applied to the vagina in a number of forms including aerosols, foams, sprays, pastes, gels, jellies, creams, suppositories, tablets, pessaries, tampons, devices such as vaginal rings, etc. They can be in the form of immediate release or controlled release. Foams, creams and gels are preferred forms. Compositions suitable for application to the vagina are disclosed in U.S. Pat. Nos. 2,149,240, 2,330,846, 2,436,184, 2,467,884, 2,541,103, 2,623,839, 2,623,841, 3,062,715, 3,067,743, 3,108,043, 3,174,900, 3,244,589, 4,093,730, 4,187,286, 4,283,325, 4,321,277, 4,368,186, 4,371,518, 4,389,330, 4,415,585, and 4,551,148, which are incorporated herein by reference, and the present method may be carried out by applying the pharmaceutical composition to the vagina in the form of such a composition.

In a particularly preferred embodiment, the pharmaceutical composition is topically applied to the vagina. Typically, the topical application is carried out prior to the beginning of vaginal intercourse, suitably 0 to 60 minutes, preferably 0 to 5 minutes, prior to the beginning of vaginal intercourse. The application may be carried out into and around the vagina and vaginal area (e.g., the individual anatomical parts, such as, labia majora, labia minora, clitoris, etc.) of a female.

The composition of the invention containing the compound of formula (I) may be applied to the vagina in any conventional manner. Suitable devices for applying the composition to the vagina are disclosed in U.S. Pat. Nos. 3,826,828, 4,108,309, 4,360,013, and 4,589,880, which are incorporated herein by reference.

Pharmaceutical creams, as known in the art, are viscous liquid or semisolid emulsions, either oil- in- water or water- in-oil. Cream bases are water- washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant.

As defined herein, a "vaginal cream" is a semi-solid preparation suitable for application to the vaginal tract. Various classes of excipients or vehicles can be used in the vaginal cream and are known to those in the art. The excipients comprise materials of naturally occurring or synthetic origin that do not adversely affect the components of the formulation. Suitable carriers for use herein include but are not limited to purified water, white soft paraffin, mucoadhesive polymers, liquid paraffin, polysorbate 60, sorbitan stearate silicone, waxes, petroleum, jelly, polyethylene glycol, and a variety of other materials, depending on the specific type of formulation used.

A preferred class of bioadhesive gelling polymers to be used in this invention is that comprised of acrylic acid polymers crosslinked with allyl sucrose or allyl ethers of pentaerythritol, commercially available with the name Carbopol (Carbomer) from B.F.Goodrich Chemical Co. Carbopol 934P and 97 IP are usually considered the ideal candidate for vaginal administration.

Another preferred class of bioadhesive gelling polymer to be used in the present invention is that comprised of acrylic acid polymers crosslinked with divinyl glycol, commercially available with the trademark Noveon AA-1 Polycarbophil USP (Polycarbophil AA1).

For example, suitable vehicle bases include, but are not limited to, hydrocarbon bases or oleaginous bases, absorption bases, water-removable bases and water-soluble bases. In some embodiments, the vehicle base is non- irritating, non-staining, stable, non-pH dependent and/or compatible with the compound of formula (I).

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of the active ingredient with the softened or melted carrier(s) followed by chilling and shaping in moulds.

In another embodiment, the present invention involves topical administration of the composition to the anus. The composition administered to the anus is suitably a foam, cream, jelly, etc., such as those described above with regard to vaginal application. In the case of anal application, it may be preferred to use an applicator which distributes the composition substantially evenly throughout the anus. For example, a suitable applicator is a tube 2.5 to 25 cm, preferably 5 to 10 cm, in length having holes distributed regularly along its length.

In another embodiment, the present method may be carried out by applying the pharmaceutical composition orally. Oral application is suitably carried out by applying a composition which is in the form of a mouthwash or gargle. Oral application is especially preferred to prevent infection during dental procedures. Suitably, the composition is applied just prior to the beginning of the dental procedure and periodically throughout the procedure. Formulations suitable for topical administration in the mouth include lozenges comprising the active ingredient in a flavored base, usually sucrose and acacia or tragacanth; pastilles comprising the active ingredient in an inert basis such as gelatin and glycerin, or sucrose and acacia; and mouthwashes comprising the active ingredient in a suitable liquid carrier.

In the case of a mouthwash or gargle, it may be preferred to include in the composition an agent which will mask the taste and/or odor of the compound of formula (I). Such agents include those flavoring agents typically found in mouthwashes and gargles, such as spearmint oil, cinnamon oil, etc.

The therapeutically effective amount of the compound or compounds of formula (I) to be administered will generally depend, among other factors, on the chosen method of administration. For this reason, the doses mentioned in this invention must only be considered as guidelines for the person skilled in the art. It is understood that animal studies may be performed to determine an appropriate dosage amount.

It is noted that when the composition is in the form of a suppository (including vaginal suppositories), the suppository will usually be 1 to 5 grams, preferably about 3 grams, and the entire suppository will be applied. A vaginal tablet will suitably be 1 to 5 grams, preferably about 2 grams, and the entire tablet will be applied. When the composition is vaginal cream, suitably 0.1 to 2 grams, preferably about 0.5 grams of the cream will be applied. When the composition is a water-soluble vaginal cream, suitably 0.1 to 2 grams, preferably about 0.6 grams, are applied. When the composition is a vaginal spray- foam, suitably 0.1 to 2 grams, preferably about 0.5 grams, of the spray-foam are applied. When the composition is an anal cream, suitably 0.1 to 2 grams, preferably about 0.5 grams of the cream is applied. When the composition is an anal spray- foam, suitably 0.1 to 2 grams, preferably about 0.5 grams of the spray- foam are applied. When the composition is a mouthwash or gargle, suitably 1 to 10 ml, preferably about 5 ml are applied.

Further, the amount of the compound or compounds of formula (I) in a dosage form must be such to achieve an effective local anal, oral or vaginal concentration, that is to say, the compound(s) of formula (I) must be present at a level sufficient to originate a microbicide effect upon administration.

In a preferred embodiment of the invention, the pharmaceutical composition comprises from about 0.1 micro g to about 300 mg of a compound of formula (I), or a pharmaceutically acceptable salt, solvate, prodrug or isomer thereof. More preferred, the composition comprises from about 1 micro g to about 30 mg of a compound of formula (I), or a pharmaceutically acceptable salt, solvate, prodrug or isomer thereof.

The present compositions may also be in the form of a time-release composition. In this embodiment, the compound of formula (I) is incorporated in a composition which will release the active ingredient at a rate which will result in an effective vaginal or anal concentration of said compound of formula (I). Time-release compositions are disclosed in Controlled Release of Pesticides and Pharmaceuticals, D. H. Lew, Ed., Plenum Press, New York, 1981; and U.S. Pat. Nos. 5,185,155; 5,248,700; 4,01 1,312; 3,887,699; 5,143,731; 3,640,741; 4,895,724; 4,795,642; Bodmeier et al, Journal of Pharmaceutical Sciences, vol. 78 (1989); Amies, Journal of Pathology and Bacteriology, vol. 77 (1959); and Pfister et al, Journal of Controlled Release, vol. 3, pp. 229-233 (1986), all of which are incorporated herein by reference.

The present compositions may also be in the form which releases the compound of formula (I) in response to some event such as vaginal or anal intercourse. For example, the composition may contain the compound of formula (I) in vesicles or liposomes, which are disrupted by the mechanical action of intercourse. Compositions comprising liposomes are described in U.S. Pat. No. 5,231,112 and Deamer and Uster, "Liposome Preparation: Methods and Mechanisms", in Liposomes, pp. 27-51 (1983); Sessa et al, J. Biol. Chem., vol. 245, pp. 3295-3300 (1970); Journal of Pharmaceutics and Pharmacology, vol. 34, pp. 473-474 (1982); and Topics in Pharmaceutical Sciences, D. D. Breimer and P. Speiser, Eds., Elsevier, New York, pp. 345-358 (1985), which are incorporated herein by reference.

It should also be realized that the present compositions may be associated with an article, such as an intrauterine device (IUD), vaginal diaphragm, vaginal ring, vaginal sponge, pessary, condom, etc. In the case of an IUD or diaphragm, time-release and/or mechanical-release compositions may be preferred, while in the case of condoms, mechanical-release compositions are preferred.

In another embodiment, the present invention provides novel articles, which are useful for the prevention of HIV infection. In particular, the present articles are those which release the compound of formula (I) when placed on an appropriate body part or in an appropriate body cavity. Thus, the present invention provides IUDs, vaginal diaphragms, vaginal sponges, pessaries, or condoms which contain or are associated with a compound of formula (I).

Thus, the present article may be an IUD which contains one or more compounds of formula (I). Suitable ILTDs are disclosed in U.S. Pat. Nos. 3,888,975 and 4,283,325 which are incorporated herein by reference. The present article may be an intravaginal sponge which comprises and releases, in a time-controlled fashion, the compound of formula (I). Intravaginal sponges are disclosed in U.S. Pat. Nos. 3,916,898 and 4,360,013, which are incorporated herein by reference. The present article may also be a vaginal dispenser, which releases the compound of formula (I). Vaginal dispensers are disclosed in U.S. Pat. No. 4,961,931, which is incorporated herein by reference.

The present article may also be a condom which is coated with a compound of formula (I). In a preferred embodiment, the condom is coated with a lubricant or penetration enhancing agent which comprises a compound of formula (I) and an spermicide, which is optionally selected from benzalkonium chloride, benzethonium chloride, cetyl pyridinium chloride, methylbenzethonium chloride, tetra-decyltrimethyl ammonium bromide, benzalkonium bromide, monylphenyl ethers, lauryl ethers, and octoxynols. However, it is recommended that use of a condom should be associated with use of an appropriate lubricating agent, i.e. one that does not degrade the mechanical strength properties of the condom and that does not increase its porosity due to the latex being attacked. For example, EP-A-0 457 127 describes a lubricant based on silicone oil for treating the latex of condoms, EP-A-0 475 664 describes a lubricating composition and use thereof with condoms, and FR-A-2 666 587 describes a lubricant comprising polydimethylsiloxane. Other lubricants and penetration enhancing agents are described in U.S. Pat. Nos. 4,537,776; 4,552,872; 4,557,934; 4,130,667, 3,989,816; 4,017,641; 4,954,487; 5,208,031; and 4,499,154, which are incorporated herein by reference.

### Microbiocide compositions of the invention

The miRNAs forming part of the profile according to the present invention are capable of discriminating HIV controller patients from patients which suffer viral progression. Thus, the invention also contemplates the use of the miRNAs for increasing the antiviral activity of anti-HIV agents. Thus, in another aspect, the invention relates to a microbiocide composition or kit-of-parts comprising
(i) A miRNAs selected from the group consisting of miR-27a, miR-27b, miR-29b and miR-221 or a polynucleotide encoding said miRNA or a polynucleotide encoding said miRNA and
(ii) an anti-HIV agent.

The term "microbiocide", as used herein, refers to a compound capable of killing, inhibiting the growth of, or controlling the growth of HIV. The microbiocide compositions according to the invention are useful for the treatment or prevention of diseases caused by HIV infection.

The term "composition", as used herein, relates to a mixture of ingredients.

As used herein, the term "kit-of-parts" shall encompass an entity of physically separated components, which are intended for individual use, but in functional relation to each other.

The term "miRNA" and the specific miRNAs "miR-27a", "miR-27b", "miR-29b", "miR-125", "miR-146", "miR-155" and "miR-221" have been described above in detail.

The term "polynucleotide encoding a miRNA" has been described in detail above.

The term "anti-HIV agent", "HIV-inhibiting agent" and "HIV antiviral agent" as used herein, refers to any compound or a pharmaceutically acceptable salt thereof which is capable of inhibiting the replication of HIV in a cell, such as a cell in a mammal or which is effective in treating, preventing, or delaying the onset or progression of HIV infection or AIDS and/or diseases or conditions arising therefrom or associated therewith. Suitable anti-HIV agents for use according to the present invention include, without limitation, HIV protease inhibitors, a HIV reverse transcriptases, HIV entry inhibitors and HIV immunogens.

The term "protease inhibitor" as used herein means inhibitors of the HIV-1 protease, an enzyme required for the proteolytic cleavage of viral polyprotein precursors (e.g. viral GAG and GAG Pol polyproteins), into the individual functional proteins found in infectious HIV-1. Suitable protease inhibitors that can be combined with the miRNAs or polynucleotides encoding miRNAs according to the invention is selected from the group consisting of ritonavir, lopinavir, saquinavir, amprenavir, fosamprenavir, nelfmavir, tipranavir, indinavir, atazanavir, TMC-126, darunavir, mozenavir (DMP-450), JE-2147 (AG1776), L-756423, KNI-272, DPC-681, DPC-684, telinavir (SC-52151), BMS 186318, droxinavir (SC- 55389a), DMP-323, KNI-227, 1-[(2-hydroxyethoxy)methyl]-6-(phenylthio)-thymine, AG-1859, RO-033-4649, R-944, DMP-850, DMP-851, and brecanavir (GW640385). Preferred protease inhibitors for use in combination with a compound of the present invention include saquinavir, ritonavir, indinavir, nelfhavir, amprenavir, lopinavir, atazanavir, darunavir, brecanavir, fosamprenavir, and tipranavir. Particularly useful such combinations include, for example, AZT+3TC; TDF+3TC; TDF+FTC; ABC+3TC; and Abacavir+3TC.

The term "reverse transcriptase inhibitors" as used herein, refer to any compound which inhibits the activity of HIV-1 reverse transcriptase, the enzyme which catalyzes the conversion of viral genomic HIV-1 RNA into proviral HIV-1 DNA. Suitable reverse transcriptase inhibitors for use in the compositions according to the present invention is one or more compounds selected from the group consisting of emtricitabine, capravirine, tenofovir, lamivudine, zalcitabine, delavirdine, nevirapine, didanosine, stavudine, abacavir, alovudine, zidovudine, racemic emtricitabine, apricitabine, emivirine, elvucitabine, TMC-278, DPC-083, amdoxovir, (-)-beta-D-2,6- diaminopurine dioxolane, MIV-210 (FLG), DFC (dexelvucitabine), dioxolane thymidine, Calanolide A, etravirine (TMC-125), L697639, atevirdine (U87201E), MIV- 150, GSK-695634, GSK-678248, TMC-278, KP1461, KP-1212, lodenosine (FddA), 5- [(3,5-dichlorophenyl)thio]-4-isopropyl-1-(4-pyridylmethyl)imidazole-2 -methanol carbamic acid, (-)-I²-D-2,6-diaminopurine dioxolane, AVX-754, BCH-13520, BMS- 56190 ((4S)-6-chloro-4-[(lE)-cyclopropylethenyl]-3,- 4-dihydro-4-trifluoromethyl-2 (1 H )-quinazolinone), TMC-120, and L697639, where the compounds are present in amounts effective for treatment of HIV when used in a combination therapy.

The term "viral entry inhibitor", as used herein, refers to any compound capable of interfering with the entry of viruses into cells. In some embodiments, the viral entry inhibitor is a fusion inhibitor, a CD4 receptor binding inhibitor, is a CD4 mimic or a gp120 mimic. In some further embodiments, the viral entry inhibitor is a gp41 antagonist, a CD4 monoclonal antibody or a CCR5 antagonist, including CCR5 antagonist sub-classes such as, for example, zinc finger inhibitors. In yet another embodiment, the viral entry inhibitor is a CXCR4 co-receptor antagonist.

The term "HIV immunogen", as used herein, refer to a protein or peptide antigen derived from HIV that is capable of generating an immune response in a subject. HIV immunogens for use according to the present invention may be selected from any HIV isolate (e.g., any primary or cultured HIV- 1, HIV-2, and / or HIV-3 isolate, strain, or clade). As is well-known in the art, HIV isolates are now classified info discrete genetic subtypes. HIV-1 is known to comprise at least ten subtypes (A1, A2, A3, A4, B, C, D, E, PL F2, G, H, j and K) (Taylor et al, MEJM. 359(18): 1965-1966 (2008)}. HIV-2 is known to include at least five subtypes (A, B, C, D, and E). Subtype B has been associated with the HIV epidemic in homosexual men and intravenous drug users worldwide. Most HIV-1 immunogens, laboratory adapted isolates, reagents and mapped epitopes belong to subtype B. In sub-Saharan Africa, India, and China, areas where the incidence of new HIV infections is high, HIV-1 subtype B accounts for only a small minority of infections, and subtype HIV-1 C appears to be the most common infecting subtype. Thus, in certain embodiments, it may be preferable to select immunogens from particular subtypes (e.g., HIV- 1 subtypes B and / or C), It may be desirable to include immunogens from multiple HIV subtypes (e.g., HIV-1 subtypes B and C HIV-2 subtypes A and B, or a combination of HIV- 1, HIV-2, and/or HIV-3 subtypes) in a single immunological composition.

Suitable HIV immunogens include HIV envelope (env; e.g., NCBI Ref. Seq. NPJ357856), gag (e.g., p6, p7, p17, p24, GenBank AAD39400J), the protease encoded by pol (e.g., UniProt P03366), nef (e.g.. fenBank -CAA4I 585J Shugars, et al J. ViroL Aug. 1993,. 4639-4650 {1993}}, as well as variants, derivatives, and fusion proteins thereof as described by, for example, Gomez et al. Vaccine, Vol. 25, pp. 1 69-1 92 (2007). Immunogens (e.g., env and pol) may be combined as desired. Immunogens from different HIV isolates. Suitable strains and combinations may be selected by the skilled artisan as desired.

The term "immunogenic composition" refers to a composition that elicits an immune response which produces antibodies or cell-mediated immune responses against a specific immunogen. As used herein, "immune response" refers to a response made by the immune system of an organism to a substance, which includes but is not limited to foreign or self proteins. Particularly, "immune response" refers to a CD8-positive T-cell mediated immune response to HIV infection. There are three general types of "immune response" including, but not limited to mucosal, humoral, and cellular "immune responses. "

An immune response to HIV may be measured by measuring anyone of viral load, T-cell proliferation, T-cell survival, cytokine secretion by T-cells, and/or an increase in the production of antigen-specific antibodies (antibody concentration), etc.

Additionally, the compositions according to the present invention may further comprise an antiretroviral agent selected from the group consisting of vaccines, gene therapy treatments, cytokines, TAT inhibitors, and imrnunomodulators in amounts effective for treatment of HIV when used in a combination therapy.

Additionally, the compositions according to the present invention may further comprise an antiinfective agent selected from the group consisting of antifungals, antibacterials, anti-neoplasties, anti-protozoals, DNA polymerase inhibitors, DNA synthesis inhibitors, anti-HIV antibodies, HIV antisense drugs, IL-2 agonists, α-glucosidase inhibitors, purine nucleoside phosphorylase inhibitors, apoptosis agonists, apoptosis inhibitors, and cholinesterase inhibitors, where the compounds are present in amounts effective for treatment of HIV when used in a combination therapy.

Additionally, the compositions according to the present invention may further comprise an immunomodulator, which is selected from the group consisting of pentamidine isethionate, autologous CD8+ infusion, γ-interferon immunoglobulins, thymic peptides, IGF-I, anti-Leu3A, auto vaccination, biostimulation, extracorporeal photophoresis, cyclosporin, rapamycin, FK-565, FK-506, GCSF, GM-CSF, hyperthermia, isopinosine, rVIG, HIVIG, passive immunotherapy and polio vaccine hyperimmunization, where the compounds are present in amounts effective for treatment of HIV when used in a combination therapy.

In another embodiment, the invention relates to a method for the treatment of a disease caused by HIV infection in a subject in need thereof comprising the administration to said subject of a composition or kit-of-parts according to the present invention.

The person skilled in the art shall understand, in the context of the present invention, that the medicament for the combined administration of a miRNA or polynucleotide encoding a miRNA and the anti-HIV agent or agents.

"Combined administration" is understood to mean that the compounds which form part of the kit-of-parts according to the invention may be administered jointly or separately, simultaneously, at the same time or sequentially in the treatment of the pathologies previously mentioned in any order. For example, the administration of the miRNA or the polynucleotide encoding said miRNA may be performed first, followed by the administration of one or more anti-HIV agents; or the administration of the miRNA or the polynucleotide encoding said miRNA may be performed at the end, preceded by the administration of one or more anti-HIV agents; or the administration of the miRNA or the polynucleotide encoding said miRNA may be performed at the same time as the administration of one or more anti-HIV agents.

The compositions or kit-of-parts of the invention may be administered using any suitable route including intravascular, intratumoral, intracranial, intraperitoneal, intrasplenic, intramuscular, subretinal, subcutaneous, mucosal, topical and oral route. In a preferred embodiment, the composition or kit-of-parts is administered topically.

The person skilled in the art shall understand, in the context of the present invention, that the medicament for the combined administration of a miRNA or polynucleotide encoding said miRNA and the anti-HIV agent may be prepared as a single dosing form or in separate dosing forms.

"Combined administration" is understood to mean that the compounds which form part of the kit-of-parts according to the invention may be administered jointly or separately, simultaneously, at the same time or sequentially in the treatment of the pathologies previously mentioned in any order. For example, the administration of the miRNA or the polynucleotide encoding a miRNA may be performed first, followed by the administration of one or more therapeutic agents useful in the treatment of diseases caused by HIV infection; or the administration of the miRNA or the polynucleotide encoding a miRNA may be performed at the end, preceded by the administration of one or more therapeutic agents useful in the treatment of diseases caused by HIV infection; or the administration of the miRNA or the polynucleotide encoding a miRNA may be performed at the same time as the administration of one or more therapeutic agents useful in the treatment diseases caused by HIV infection.

The compositions or kit-of-parts of the invention may be administered using any suitable route including intravascular, intratumoral, intracranial, intraperitoneal, intrasplenic, intramuscular, subretinal, subcutaneous, mucosal, topical and oral route. In a preferred embodiment, the composition or kit-of-parts is administered topically.

Suitable routes of administration of the compositions or kit-of-parts according to the invention have been described above in detain in the context of the medical uses of the miRNAs and polynucleotides encoding thereof and are equally applicable to the compositions or kit-of-parts of the invention.

### miRNA signature-specific microarrays, oligonucleotide primers, and kits

Following the identification of an miRNA signature according to the methods of the present invention, microarrays can be constructed which contain oligonucleotide probes that specifically recognize the miRNAs identified as comprising the miRNA signature. Thus, in another aspect, the invention relates to a kit comprising reagents adequate for the determination of the expression levels of one or more miRNAs selected from the group consisting of A kit comprising reagents adequate for the determination of the expression levels of one or more miRNAs selected from the group consisting of miR-27a, miR-27b, miR-29b, miR-221 miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-Sp, miR-191 and/or miR-197.

In a preferred embodiment, the kit comprises reagents for the determination of the expression levels of the miRNAs selected from the group consisting of the miR-27a, miR-27b, miR-29b and miR-221.

Such microarrays are useful in the diagnostic, therapeutic, and screening assays described herein. Microarrays containing probes recognizing the subset of miRNAs comprising an miRNA signature have the advantages of being cost-effective to produce and simpler to analyze, as they do not include extraneous probes that are not relevant to the detection of, i.e., a virus, the replication stage of a virus, or the disease or pathological state of a subject infected with a virus. In one embodiment, such a microarray also contains oligonucleotide probes specific for RNAs suitable for normalization purposes. Such probes are well known in the art, and include, for example, probes recognizing 18S ribosomal RNA, mRNA encoding GAPDH, mRNA encoding beta-actin, etc.

In a preferred embodiment, the reagents adequate for the determination of the expression levels of said one or more miRNAs comprise at least 50% of the total amount of reagents forming the kit.

In further embodiments, the reagents adequate for the determination of the expression levels of one or more genes comprise at least 55% at least 60%, at least 65%, at least 70%, at least 75%, at least 80%, at least 90%, at least 95%, at least 96%, at least 97%, at least 98% or at least 99% of the total amount of reagents forming the kit.

In a related aspect, the present invention provides a set of one or more pairs of oligonucleotide primers designed to specifically amplify the miRNAs of an miRNA signature identified using the methods of the invention in a reverse-transcriptase polymerase chain reaction assay, preferably a real-time quantitative reverse-transcriptase polymerase chain reaction assay. Quantitative reverse-transcriptase polymerase chain reaction can provide a cost-effective alternative to microarray analysis when the number of miRNAs identified as part of a miRNA signature is relatively small.

The present invention further provides a kit containing a microarray that has miRNA-specific probe oligonucleotides which specifically recognize miRNAs that were identified as part of an miRNA signature using methods of the invention. The microarrays contained in the kit may also include probe oligonucleotides which specifically recognize RNAs suitable for normalization purposes. In certain embodiments the kit further contains a control sample. This sample can be derived from a control or normal cell, tissue, or subject, e.g., one that exhibits, for example, normal traits. In additional embodiments, the kits of the invention contain instructions for use. In an alternative aspect, the present invention provides a kit containing a set of one or more pairs of oligonucleotide primers designed to specifically amplify the miRNAs of an miRNA signature identified using the methods of the invention in a reverse-transcriptase polymerase chain reaction assay, preferably a real-time quantitative reverse-transcriptase polymerase chain reaction assay.

The expression "reagent which allows determining the expression level of a gene" means a compound or set of compounds that allows determining the expression level of a gene both by means of the determination of the level of mRNA or by means of the determination of the level of protein. Thus, reagents of the first type include probes capable of specifically hybridizing with the mRNAs encoded by said genes. Reagents of the second type include compounds that bind specifically with the proteins encoded by the marker genes and preferably include antibodies, although they can be specific aptamers.

In a particular embodiment of the kit of the invention, the reagents of the kit are nucleic acids which are capable of specifically detecting the mRNA level of the genes mentioned above and/or the level of proteins encoded by one or more of the genes mentioned above. Nucleic acids capable of specifically hybridizing with the genes mentioned above can be one or more pairs of primer oligonucleotides for the specific amplification of fragments of the mRNAs (or of their corresponding cDNAs) of said genes.

In a preferred embodiment, the first component of the kit of the invention comprises a probe which can specifically hybridize to the genes mentioned above.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50 °C.; (2) employ during hybridization a denaturing agent, such as formamide, for example, 50% (v/v) formamide with 0.1% bovine serum albumin / 0.1% Ficoll/0.1% polyvinylpyrrolidone/50 mM sodium phosphate buffer at pH 6.5 with 750 mM sodium chloride, 75 mM sodium citrate at 42 °C.; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C. in 0.2 x SSC (sodium chloride/sodium citrate) and 50% formamide, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37 °C. in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50 °C. The skilled artisan will recognize how to adjust the temperature, ionic strength, etc. as necessary to accommodate factors such as probe length and the like.

This invention is further illustrated by the following example which should not be construed as limiting. The contents of all references, patents and published patent applications cited throughout this application are incorporated herein by reference.

### EXAMPLE

### Materials and methods

### Research subjects and sample processing

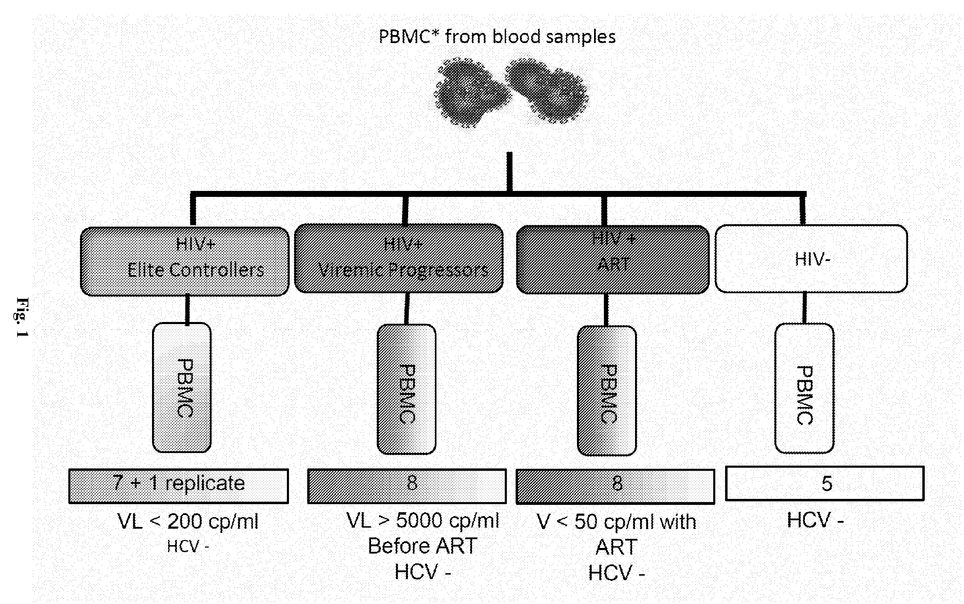

Blood samples were obtained from HIV infected and non infected donors without hepatitis C co-infection, from HIV Unit of Hospital Clinic of Barcelona. All donors signed an agreement to voluntary enrol in this study. PBMC from four cohorts were taken from: elite controllers (EC) ( n= 8, viral load < 50 cp/ml for more than 1 years , 3 titrations per year and CD4+ cells > 450 cells / ul ), viremic progressors (VP) before HAART treatment (n=8 , mean plasma viral load = 15.000 cp/ml, ART HIV infected patients (ART) (n=8, viral load <200 cp/ml with treatment) and HIV negative individuals (HIV-) (n=5) (Figure 1). The viral load (cp/ml) and expression levels of HLA-A. HLA-B and HLA-C for the different patients are provided in Table 1.

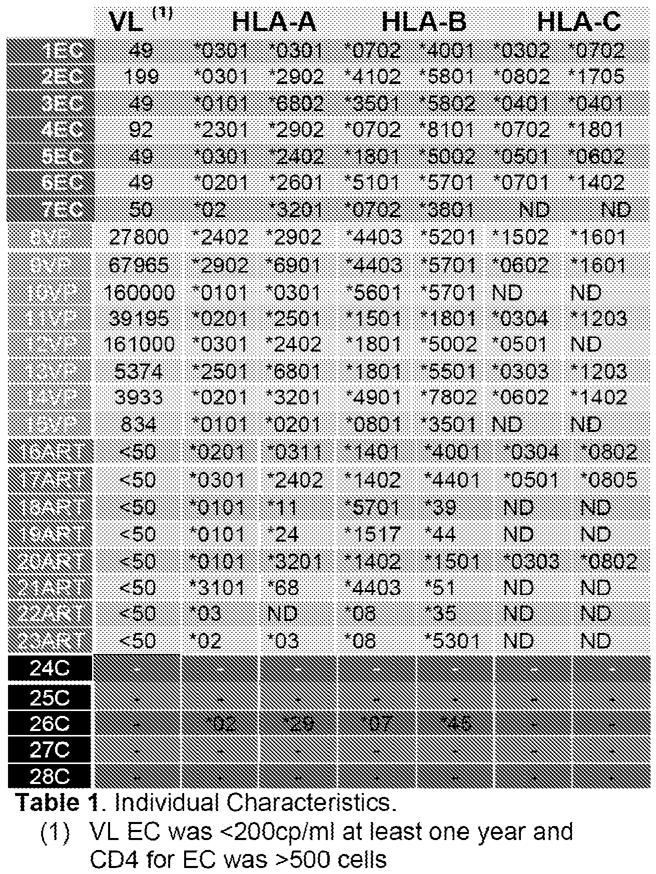

Extracted plasma blood mononuclear cells (PBMCs) were cultured in RPMI modified media and activated with phyto-hemagglutinin 10 ul / ml during 48 hours. Cells pellets were obtained after centrifugation ( x g , x mn) and washed twice in sterile PBS 1X. Total RNA including small RNAs were extracted with MirVana kit (Ambion) according to the manufacturer recommendations. All RNA samples were quantified using nanodrop spectrophotometer and qualified with Agilent 2100 bioanalyzer using total RNA nano chip. Only samples with a RNA integrity number (RIN) > 7 were processed. 350 ng of total RNA were simultaneously reverse transcribed and preamplified according to the Megaplex pools protocol (Applied biosystems). The pre-amplified products were processed on TaqMan® Array Human MicroRNA Set Cards v2.0 (Applied Biosystems) and quantitative PCR was run on ABI 7900 (Applied biosystems) according to the protocol.

### Statistical analysis

For each sample Ct (cycle threshold) values were calculated using RealTime StatMiner® Software (Integromics) after importation of the quantification values from sds 2.4 software (Applied Biosystems). Each Ct was normalised by substraction of the mean value of all the expressed miRNAs of the same sample (global mean normalisation) according to data published before (Pieter Mestdagh et al.). miRNAs with a Ct value >35 were considered as non expressed and changed to a Ct value of 40. miRNAs that were not expressed in more than 25 % of each cohort were excluded from the analysis. Finally a set of 168 expressed miRNAs genes was selected for the analysis. A non parametric Mann-Whitney U test was run in TMEV software V4.5 (Saeed AI et al., TM4: a free, open-source system for microarray data management and analysis. Biotechniques. 2003 Feb;34(2):374-8.) with a estimated false discovery rate (FDR) of 5%, between the groups elite controllers (EC) and viremic progressors (VP). FDR is used to control type I errors using Benjamini-Hochberg correction test.

Fold change expression relative to HIV negative group (HIV-) for the differentially expressed miRNAs was calculated by comparative deltaCt method. Mean Ct normalised expression values of each miRNAs was calculated for EC and VP group and subtracted to the mean normalised Ct value of the reference group (HIV -). Fold change of each group relative to reference group was then calculated according to the 2 ^{-ΔCT} formula.

### RESULTS

The expression of the human set of miRNAs (Applied Biosystems , MicroRNA Set Cards v2.0) was evaluated by quantitative PCR from PBMCs of 4 groups of individuals (Figure 1).

After adequate normalisation steps a statistic analysis of significatively differentially expressed human miRNAs was performed between elite controllers (EC) and viremic progressors (VP). Non parametric Mann-Whitney U test set to a false discovery rate (FDR) of 5% provided a list of 23 candidate miRNAs (Table 2).

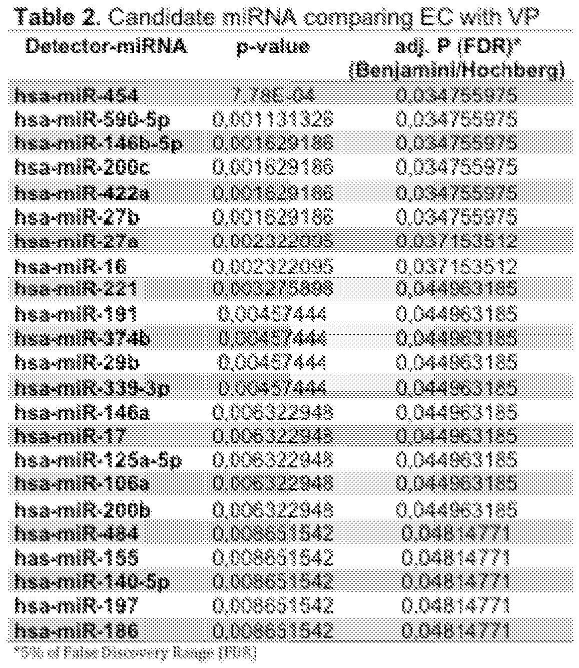

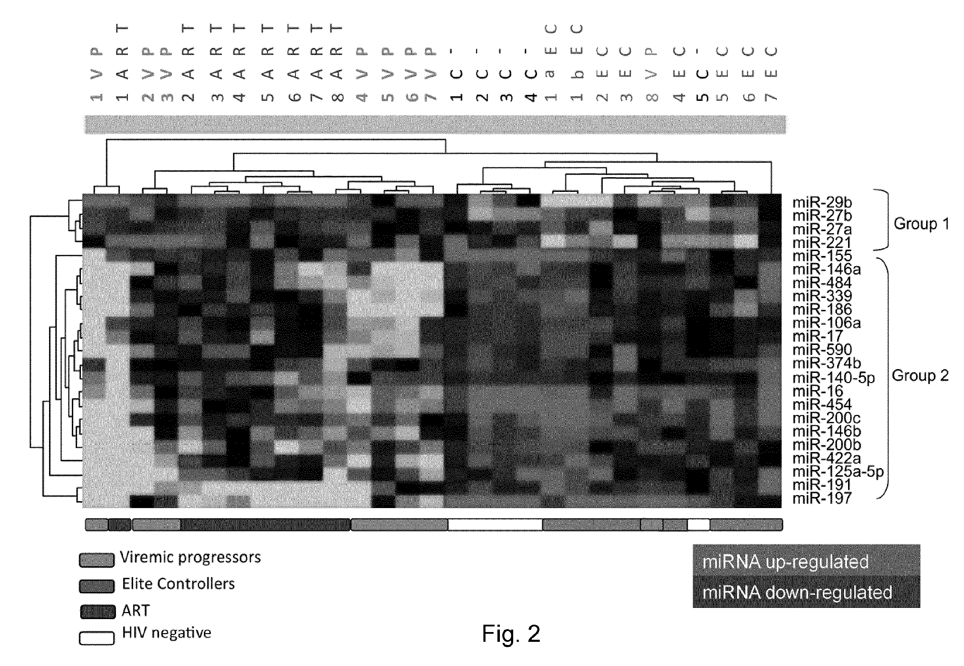

A hierarchical clustering (average linkage clustering constructed on Euclidian distances) of the candidates miRNAs of the 4 groups (EC; VP; ART; HIV Neg) of the study segregated the population in two separate blocks (Figure 2). The first block was more heterogeneous and included EC and HIV negative with no significant differences on miRNA expression and the second block included VP and ART patients as a subgroup of this second block. One of the VP patients (8VP) is mixed between patients of the second block. Two miRNA expression patterns segregate between those two blocks; a set of 4 miRNAs are under expressed at the VP group and overexpressed in the EC (miR-221, 27a, 27b and 29b, group 1 on fig.2), whereas a set of 19 miRNAs (miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-Sp, miR-191 and/or miR-197) are overexpressed at the VP group and under expressed at the EC (group 2 on Fig.2).

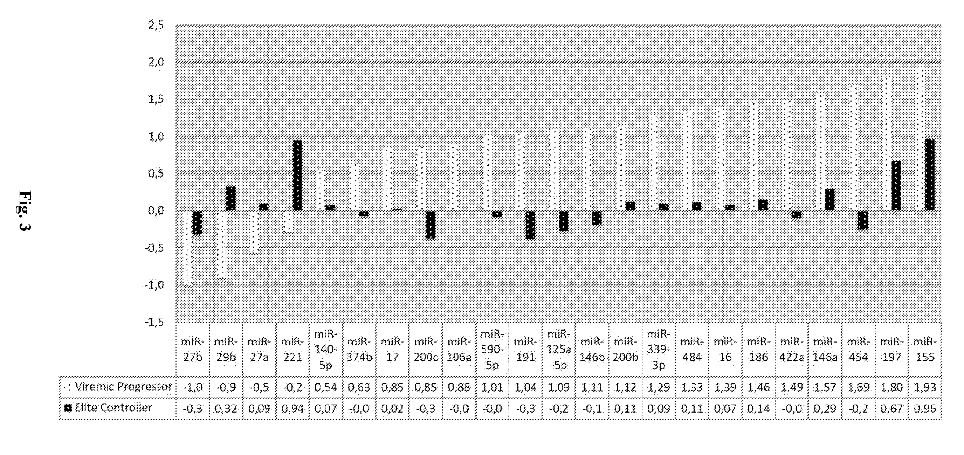

For selected miRNAs, results were expressed as the mean fold change of EC and VP relative to reference HIV negative group by the ΔΔCt method. The variation of this fold change is represented on a log2 scale in Fig.3A. On this graphical representation, close to "0" values are miRNAs that tend to have a similar expression compared to HIV negative group whereas positive and negative values are miRNAs that are over or under expressed compared to HIV negative group.

Concerning EC group, most of the miRNAs (miR-221, miR-197 and miR-155 excluded) values tend to have an expression value similar to HIV negative individuals. The fold change (mir-221, 197 and 155 excluded) approximatively ranging from 1 to 1.3 up and below. miR-221 and miR-155 are the only ones to be clearly more up-regulated in this group (2 up-fold change).

Concerning the group of VP, 19 out of the 23 miRNAs are clearly up-regulated, h ranging from approximately 1.5 to 3.3 fold change (fig.3A). Only 4 of them are down-regulated (from 1.2 to 2 down fold-change).

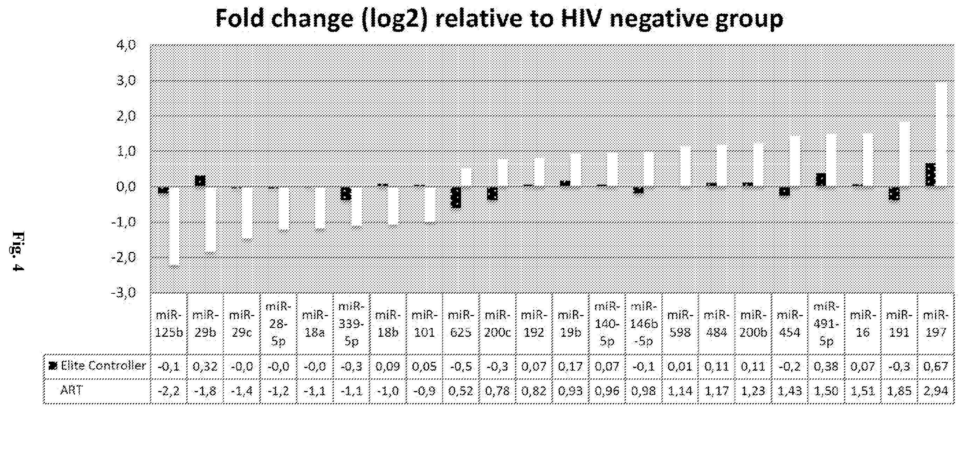

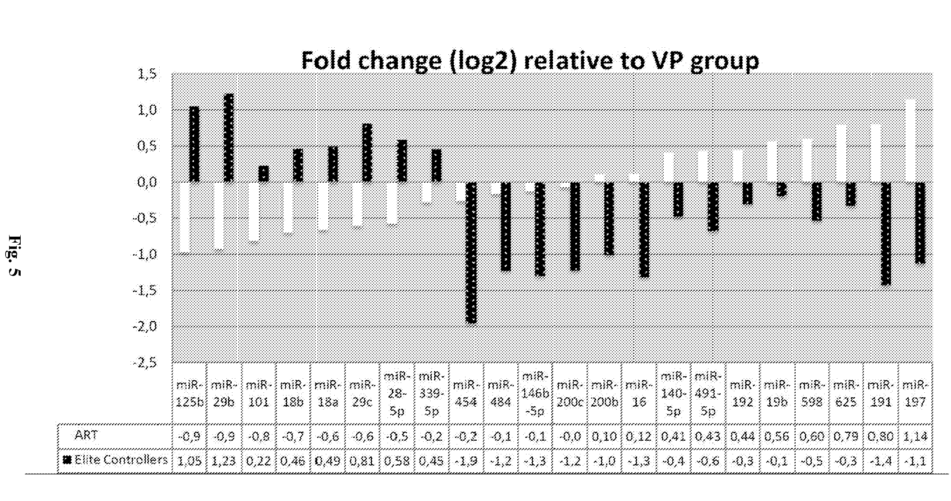

A comparison between EC and ART was performed in parallel and a total of 22 candidate miRNA were obtained (Table 2). Again the fold change was calculated in EC and ART relative to HIV Neg and VP respectively (Figures 4 and 5).

A more than 2 fold-change of miRNA expression of ART (14 up-regulated and 8 down-regulated) was observed relative to HIV neg group (Figure 4). However comparing EC and ART relative to VP, 10 miRNA (two up-regulated: miR-125 and miR-29b and eight down-regulated miR-454, miR-484, miR-146b, miR-200c, miR-200b, miR-16, miR-191, miR-197) showed more than 2 fold-change while other 12 miRNA (6 up-regulated: miR-101, miR-18a, miR-18b, miR-29c, miR-28-5p, miR-339-5p and 6 down-regulated: miR-140-5, miR-491-5p, miR-192, miR-19b, miR-598, miR-625) showed a fold-change lower than 2 (Figure 5).

Analyzing the differential miRNA expression level, all possible comparison between these groups were performed taking into account the possible miRNA signatures for each group related with viral load and immunogenetics. 10 out of 23 miRNA with a different expression level when EC are compared with VP are the same out of 22 miRNA when EC are compared with ART group. However, these 10 miRNA showed a different direction in expression, those that are over-expressed at the EC versus VP are under-expressed at the EC versus ART comparison and viceversa. No differential miRNA expression level was observed at EC versus HIV neg neither nor at VP versus ART or ART versus HIV Neg. Comparing HIV negative versus VP and versus ART a total of 25 and 52 miRNA were differentially expressed. 17 out of 25 were the same when EC versus VP comparison is done.

## Claims

1. A method for the identification of a controller HIV-infected patient which comprises the determination of the level of one or more miRNAs selected from the group consisting of miR-27a, miR-27b, miR-29b, miR-221 miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-Sp, miR-191 and/or miR-197 in a sample from said patient,
wherein increased expression level of one or more of the miR-27a, miR-27b, miR-29b and/or miR-221 miRNAs with respect to a reference value is indicative that the patient is a HIV controller or
wherein decreased expression levels of one or more of miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-5p, miR-191 and/or miR-197 with respect to a reference value is indicative that the patient is a HIV controller.

2. A method according to claim 1 wherein the controller is an elite controller.

3. A method as defined in claims 1 or 2 wherein the sample is a PBMC preparation.

4. A method as defined in any of claims 1 to 3 wherein the reference value is the expression level of the miRNA in a sample from a viremic progressor or from a HIV-infected patient undergoing ART.

5. A miRNAs selected from the group consisting of miR-27a, miR-27b, miR-29b, miR-125, miR-146, miR-155 and miR-221 for use in the treatment or prevention of a disease caused by HIV.

6. A microbiocide composition or kit-of-parts comprising
(i) A miRNAs selected from the group consisting of miR-27a, miR-27b, miR-29b, miR-125, miR-146, miR-155 and miR-221 or a polynucleotide encoding said miRNA and
(ii) an anti-HIV agent.

7. A composition or kit-of-parts as defined in claim 6 for use in medicine.

8. A composition or kit-of-parts as defined in claim 6 for use in the treatment of a disease caused by HIV infection.

9. A kit comprising reagents adequate for the determination of the expression levels of one or more miRNAs selected from the group consisting of miR-27a, miR-27b, miR-29b, miR-221 miR-155, miR-146a, miR-484, miR-339, miR-186, miR-106a, miR-17, miR-590, miR-374b, miR-140-Sp, miR-16, miR-454, miR-200c, miR-146b, miR-200b, miR-422a, miR-125a-Sp, miR-191 and/or miR-197.

10. A kit as defined in claims 9 or 10 wherein the reagents adequate for the determination of the expression levels of said one or more of said miRNAs are oligonucleotides that hybridise specifically to said one or more of said miRNAs.


SEQUENCE LISTING

<110>  Laboratorios del Dr. Esteve, S.A.
       Fundació Privada Institut de Recerca de la SIDA-Caixa

<120>  METHODS AND REAGENTS FOR EFFICIENT CONTROL OF HIV PROGRESSION

<130>  P7393EP00

<160>  23

<170>  PatentIn version 3.5

<210>  1
<211>  23
<212>  RNA
<213>  H.sapiens

<400>  1
uagugcaaua uugcuuauag ggu                                              23


<210>  2
<211>  22
<212>  RNA
<213>  H.sapiens

<400>  2
gagcuuauuc auaaaagugc ag                                               22


<210>  3
<211>  22
<212>  RNA
<213>  H.sapiens

<400>  3
ugagaacuga auuccauagg cu                                               22


<210>  4
<211>  23
<212>  RNA
<213>  H.sapiens

<400>  4
uaauacugcc ggguaaugau gga                                              23


<210>  5
<211>  22
<212>  RNA
<213>  H.sapiens

<400>  5
acuggacuua gggucagaag gc                                               22


<210>  6
<211>  21
<212>  RNA
<213>  H.sapiens

<400>  6
uucacagugg cuaaguucug c                                                21

<210>  7
<211>  21
<212>  RNA
<213>  H.sapiens

<400>  7
uucacagugg cuaaguuccg c                                                21


<210>  8
<211>  22
<212>  RNA
<213>  H.sapiens

<400>  8
uagcagcacg uaaauauugg cg                                               22


<210>  9
<211>  23
<212>  RNA
<213>  H.sapiens

<400>  9
agcuacauug ucugcugggu uuc                                              23


<210>  10
<211>  23
<212>  RNA
<213>  H.sapiens

<400>  10
caacggaauc ccaaaagcag cug                                              23


<210>  11
<211>  22
<212>  RNA
<213>  H.sapiens

<400>  11
auauaauaca accugcuaag ug                                               22


<210>  12
<211>  23
<212>  RNA
<213>  H.sapiens

<400>  12
uagcaccauu ugaaaucagu guu                                              23


<210>  13
<211>  23
<212>  RNA
<213>  H.sapiens

<400>  13
ucccuguccu ccaggagcuc acg                                              23

<210>  14
<211>  22
<212>  RNA
<213>  H.sapiens

<400>  14
ugagaacuga auuccauggg uu                                                22

<210>  15
<211>  23
<212>  RNA
<213>  H.sapiens

<400>  15
caaagugcuu acagugcagg uag                                               23

<210>  16
<211>  24
<212>  RNA
<213>  H.sapiens

<400>  16
ucccugagac ccuuuaaccu guga                                              24

<210>  17
<211>  23
<212>  RNA
<213>  H.sapiens

<400>  17
aaaagugcuu acagugcagg uag                                               23

<210>  18
<211>  22
<212>  RNA
<213>  H.sapiens

<400>  18
uaauacugcc ugguaaugau ga                                                22

<210>  19
<211>  22
<212>  RNA
<213>  H.sapiens

<400>  19
ucaggcucag uccccucccg au                                                22

<210>  20
<211>  23
<212>  RNA
<213>  H.sapiens

<400>  20
uuaaugcuaa ucgugauagg ggu                                               23

<210>  21
<211>  22

<212>  RNA
<213>  H.sapiens

<400>  21
cagugguuuu acccuauggu ag                                                22

<210>  22
<211>  22
<212>  RNA
<213>  H.sapiens

<400>  22
uucaccaccu ucuccaccca gc                                                22

<210>  23
<211>  22
<212>  RNA
<213>  H.sapiens

<400>  23
caaagaauuc uccuuuuggg cu                                                22